# EUROPEAN PATENT SPECIFICATION

(11) **EP 2 488 178 B1**
(45) Date of publication and mention of the grant of the patent: **16.11.2016**
(21) Application number: 10768448.2
(22) Date of filing: 11.10.2010
(51) Int. Cl.: A61K 31/445, A61K 31/5377, A61K 35/00, A61P 35/00, A61P 35/04, A61K 31/4523

(54) **COMBINATIONS OF A PI3K INHIBITOR AND A MEK INHIBITOR**
KOMBINATIONEN AUS EINEM PI3K-NHIBITOR UND EINEM MEK-INHIBITOR
COMBINAISONS D'UN INHIBITEUR DE PI3K ET D'UN INHIBITEUR DE MEK

(30) Priority: 12.10.2009 US 250852 P
(43) Date of publication of application: 22.08.2012
(73) Proprietor: F.Hoffmann-La Roche AG, 4070 Basel (CH)
(72) Inventor: BELVIN, Marcia, South San Francisco CA 94080 (US); CHAN, Iris, T., South San Francisco CA 94080 (US); FRIEDMAN, Lori, South San Francisco CA 94080 (US); HOEFLICH, Klaus, P., South San Francisco CA 94080 (US); PRESCOTT, John, South San Francisco CA 94080 (US); WALLIN, Jeffrey, South San Francisco CA 94080 (US)
(74) Representative: Sauer, Frank
(86) International application number: PCT/EP2010/065149
(87) International publication number: WO 2011/054620

(56) References cited:
- WO-A1-2007/044515
- WO-A1-2008/070740
- WO-A2-2007/129161
- CHOO EDNA F ET AL: "PK-PD Modeling of Combination Efficacy Effect from Administration of a MEK Inhibitor, GDC-0973 and PI3K Inhibitor, GDC-0941 in A2058 Xenografts", DRUG METABOLISM REVIEWS, MARCEL DEKKER, NEW YORK, NY, US, vol. 42, no. Suppl. 1, 1 August 2010 (2010-08-01), page 268, XP009141598, ISSN: 0360-2532

## Description

The invention relates to a combination of an inhibitor of phosphatidylinositol 3-kinase (PI 3-kinase or PI3K) and an inhibitor of mitogen activated protein kinase kinase (MEK) described herein for use in treating a patient with locally advanced or metastatic solid tumors.

The mitogen-activated protein kinase (MAPK) signaling cascade transduces multiple proliferative and differentiating signals within tumor cells. Four MAPK pathways have been identified: extracellular signal-regulated kinase (ERK), c-Jun NH₂-terminal kinase (JNK), p38 kinase, and ERK5 (Johnson and Lapadat, Science 2002;298(5600):1911-2). Different extracellular signals can stimulate one or more of these pathways.

The RAS/RAF/MAPK/ERK pathway plays a major role in mediating cell growth and differenttiation in response to numerous extracellular signals. Ras-GTP activates Raf kinase, which in turn activates the MEK/ERK pathway and drives cellular proliferation (Downward, Nat Rev Cancer. 2003;3(1):11-22). To regulate cellular proliferation, activated ERKs translocate to the nucleus and regulate gene expression through the activation of several key transcription factors. Abnormal regulation of the RAS/RAF/MEK/ERK pathway contributes to uncontrolled proliferation, invasion, metastasis, angiogenesis, and diminished apoptosis.

Inhibitors of MEK would be expected to be most efficacious in tumors that are highly dependent on proliferative signals from the RAS/RAF/MEK/ERK signaling pathway. Mutation and/or overexpression of EGFR as well as mutations in the KRAS, NRAS, and BRAF oncogenes activate this pathway in many cancers. RAS is mutated in approximately 30% of all solid tumors (Well-come Trust Sanger Institute, COSMIC database). Oncogenic KRAS mutations are found with high incidence in pancreatic adenocarcinoma (90%), colorectal adenocarcinoma (30%-50%) and non-small cell lung cancer (30%) (Johnson et al., Nature 2001;410:1111-1116). Activating somatic mutations in the B-RAF oncogene, (e.g., B-RAF ^{V600E}) have been identified in a number of malignancies, with the highest incidence in malignant melanoma (60%-80%), papillary thyroid cancer (35%-70%), colorectal cancer (about 10%), and endometrial cancer (10%-20%). Cancer cells transformed by B-RAF^{V600E} are exceptionally sensitive to MEK inhibition. Therefore, MEK inhibitors may have particular clinical utility in melanoma and other tumors harboring the B-RAF^{V600E} mutation (Solit, Nature 2006; 441:424-30).

The phosphoinositide 3-kinase (PI3K) signaling pathway is a major downstream effector of receptor tyrosine kinases that stimulate cell proliferation, promote survival, and inhibit apoptosis, such as human epidermal growth factor-2 (HER2), epidermal growth factor receptor (EGFR), and insulin-like growth factor-1 receptor. Abnormal regulation of this central signaling pathway has been identified in a large number of cancer types, occurring through a variety of mechanisms. The pathway is constitutively activated by the loss of the tumor suppressor phosphatase and tensin homolog (PTEN), a phosphatase that counteracts the kinase activity of PI3K, in many tumor types (Li et al., Science 1997;275:1943-7; Steck et al., Nat Genet 1997;15:356-62). AKT, a downstream target for PI3K, is overexpressed in some tumor types (Staal, Proc Nat Acad Sci USA 1987;84(14):5034-7; Cheng et al., Proc Nat Acad Sci USA 1992;89(19):9267-71; Bella-cosa et al., Int J Cancer 1995;64(4):280-5) and has been shown to be transforming (Aoki et al., Proc Nat Acad Sci USA 1998;95(25):14950-5). Activating mutations of PI3K-α, which belongs to the class IA PI3K family, have been observed in a number of different tumor types (Bachman et al., Cancer Biol Ther 2004;3:772-5; Samuels et al., Science 2004;304:554).

These activating mutations have been shown to promote growth and invasion in cancer cells, effects that are abrogated by PI3K inhibitors. Taken together, these data provide a strong rationale for developing inhibitors of PI3K pathway signaling as a therapeutic strategy for human cancer.

WO2007/129161 relates to thienopyrimidines and the pharmaceutically acceptable salts thereof which have activity as inhibitors of PI3K with selectivity for the P110 subtype, and may be used to treat diseases and disorders arising from abnormal cell growth, function or behaviour, particularly those associated with PI3 kinase such as cancer, immune disorders, cardiovascular disease, viral infection, inflammation, metabolism/endocrine disorders and neurological disorders.

WO 2008/070740 relates to compounds, stereoisomers, geometric isomers, tautomers, solvates, metabolites and pharmaceutically acceptable salts thereof, which are useful for modulating the activity of lipid kinases including PI3K, and for treating disorders such as cancer mediated by lipid kinases.

WO 2007/044515 relates to compounds and pharmaceutically acceptable salts and solvates thereof. Such compounds are MEK inhibitors and are useful in the treatment of proliferative diseases, such as cancer. Many cancers (e.g., melanoma, colorectal, pancreatic, ovarian, NSCLC, and thyroid cancers) have a high and overlapping frequency of oncogenic mutations that activate both RAS and PI3K pathways. Furthermore, in tumor cells, inhibition of one activated pathway can result in activation of the other; therefore, inhibition of both RAS and PI3K pathways represents a new anti-cancer strategy. Thus, combined MEK and PI3K inhibition is an exciting approach to treat cancers.

The invention relates to compositions of 4-(2-(1H-indazol-4-yl)-6-((4-(methylsulfonyl)piperazin-1-yl)methyl)thieno[3,2-d]pyrimidin-4-yl)morpholine **(I),** also known as GDC-0941 (US 2008/0076768; WO 2006/046031), which inhibits PI3K, and an inhibitor of MEK described in herein for use in treating a patient with locally advanced or metastatic solid tumors.

The invention further relates to compositions of **I** and a MEK inhibitor wherein the inhibitor is [3,4-difluoro-2-(2-fluoro-4-iodo-phenylamino)-phenyl]-((S)-3-hydroxy-3-piperidin-2-yl-azetidin-1-yl)-methanone also known as GDC-0973/XL-518 (**III**).

The invention further relates to dosages of **I** and **III** which can be used in combination therapy and dosing regimes useful for practicing combination therapy with **I** and **III.**

The invention relates to a therapeutic combination of a therapeutically effective amount of a compound of formula **I,** or a pharmaceutically acceptable salt of **I,** and a therapeutically effective amount of a compound of formula **III,** or a pharmaceutically acceptable salt of **III.** 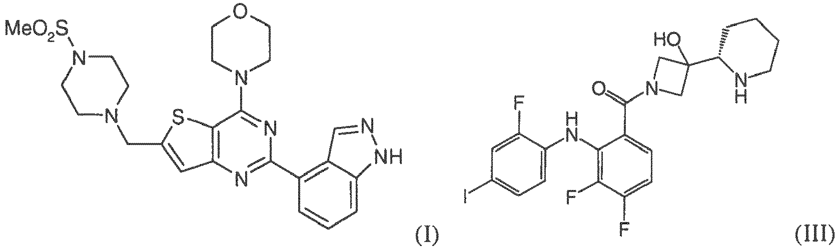 for use in treating locally advanced or metastatic solid tumors in a mammal as a combined formulation or by alternation.

The invention relates to a therapeutic formulation of a therapeutically effective amount of a compound of formula **I,** or a pharmaceutically acceptable salt of **I,** and a therapeutically effective amount of a compound of formula **III,** or a pharmaceutically acceptable salt of **III.** 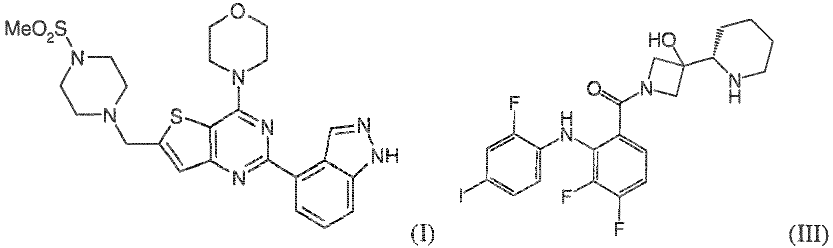 for use in treating locally advanced or metastatic solid tumors in a mammal as a combined formulation or by alternation.

In one embodiment the invention relates to the therapeutic formulation for use according the above wherein said patient is administered concurrently 80 mg, 100 mg, 130 mg or 180 mg of **I** and 20 mg, 40 mg, or 60 mg of **III.**

In one embodiment the invention relates to the therapeutic formulation for use according to the above wherein said patient is administered a compound of formula **I** in combination with a compound of formula **III** wherein said patient is on a 28 day cycle in which said patient is administered both the compound of formula **I** and the compound of formula **III** for 21 consecutive days and no compound of formula **I** or **III** for the next 7 consecutive days.

In one embodiment, the invention relates to the therapeutic formulation for use according to the above wherein said patient is administered a compound of formula **I** in combination with a compound of formula **III** wherein said patient is on a 28 day cycle in which said patient is administered both, the compound of formula **I** and the compound of formula **III** for 14 consecutive days and no compound of formula **I** or **III** for the next 14 consecutive days.

In one embodiment, the invention relates to the use of a compound of formula **I** in combination with a compound of formula **III** 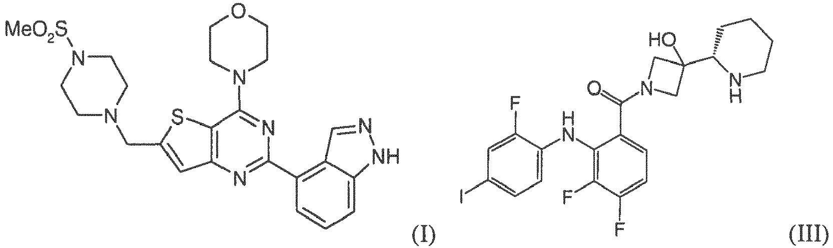 in the preparation of a medicament for the treatment of locally advanced or metastatic solid tumors.

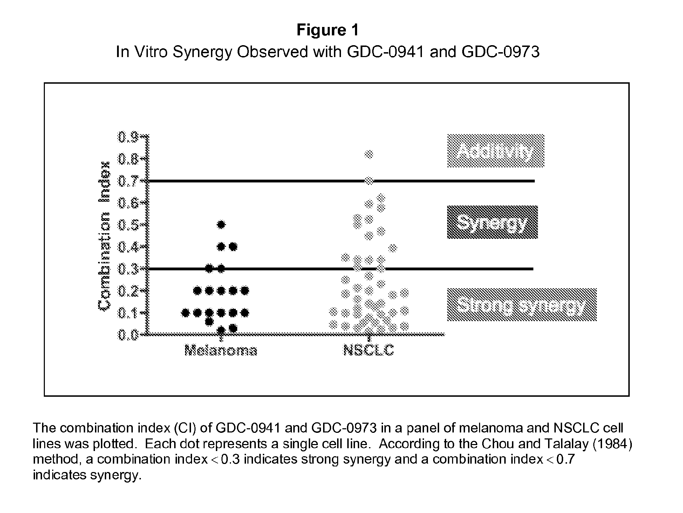

Figure 1 shows in vitro synergy observed with GDC-0941 and GDC-0973. The combination index (CI) of GDC-0941 and GDC-0973 in a panel of melanoma and NSCLC cell lines was plotted. Each dot represents a single cell line. According to the Chou and Talalay (1984) (Adv. Enz. Regul. 1984 22:27-55) method, a combination index <0.3 indicates strong synergy and a combination index <0.7 indicates synergy.

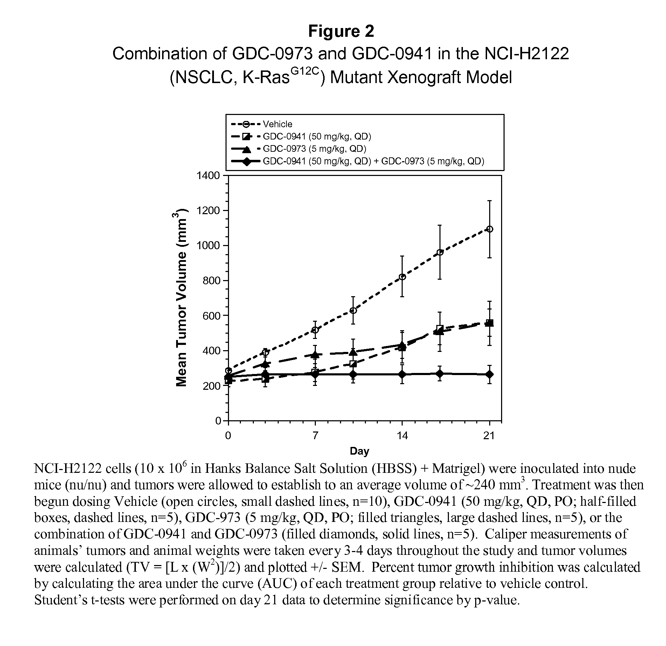

Figure 2 shows combination of GDC-0973 and GDC-0941 in the NCI-H2122 (NSCLC, K-Ras^{G12C}) mutant Xenograft model. NCI-H2122 cells (10 x 10⁶ in Hanks Balance Salt Solution (HBSS) + Matrigel) were inoculated into nude mice (nu/nu) and tumors were allowed to establish to an average volume of -240 mm³. Treatment was then begun dosing Vehicle (open circles, small dashed lines, n=10), GDC-0941 (50 mg/kg, QD, PO; half-filled boxes, dashed lines, n=5), GDC-973 (5 mg/kg, QD, PO; filled triangles, large dashed lines, n=5), or the combination of GDC-0941 and GDC-0973 (filled diamonds, solid lines, n=5). Caliper measurements of animals' tumors and animal weights were taken every 3-4 days throughout the study and tumor volumes were calculated (TV = [L x (W²)]/2) and plotted +/- SEM. Percent tumor growth inhibition was calculated by calculating the area under the curve (AUC) of each treatment group relative to vehicle control. Student's t-tests were performed on day 21 data to determine significance by p-value.

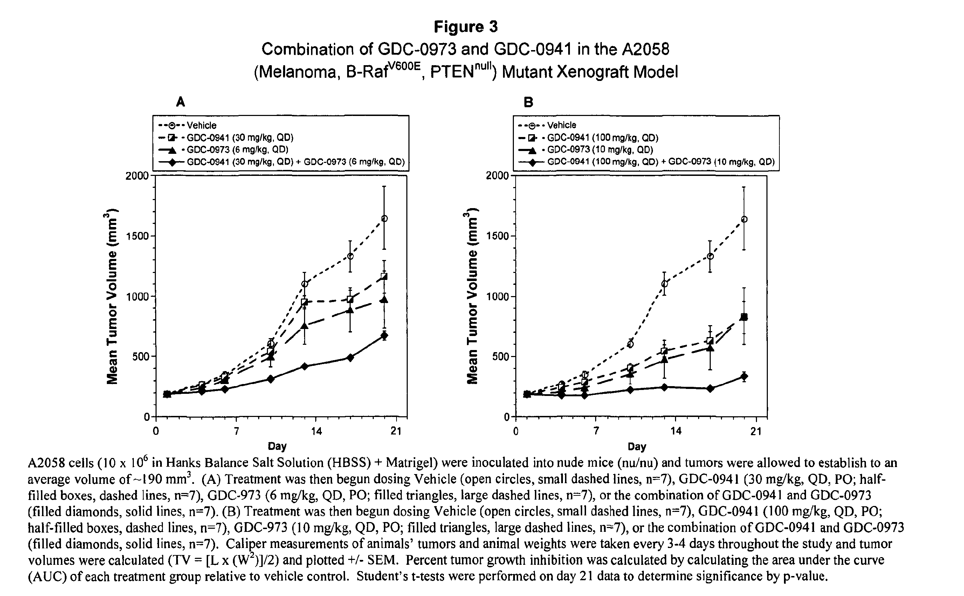

Figure 3 shows combination of GDC-0973 and GDC-0941 in the A2058 (Melanoma, B-Raf^{V600E}, PTEN^{null}) mutant Xenograft model. A2058 cells (10 x 10⁶ in Hanks Balance Salt Solution (HBSS) + Matrigel) were inoculated into nude mice (nu/nu) and tumors were allowed to establish to an average volume of -190 mm³. (A) Treatment was then begun dosing Vehicle (open circles, small dashed lines, n=7), GDC-0941 (30 mg/kg, QD, PO; half-filled boxes, dashed lines, n=7), GDC-973 (6 mg/kg, QD, PO; filled triangles, large dashed lines, n=7), or the combination of GDC-0941 and GDC-0973 (filled diamonds, solid lines, n=7). (B) Treatment was then begun dosing Vehicle (open circles, small dashed lines, n=7), GDC-0941 (100 mg/kg, QD, PO; half-filled boxes, dashed lines, n=7), GDC-0973 (10 mg/kg, QD, PO; filled triangles, large dashed lines, n=7), or the combination of GDC-0941 and GDC-0973 (filled diamonds, solid lines, n=7). Caliper measurements of animals' tumors and animal weights were taken every 3-4 days throughout the study and tumor volumes were calculated (TV = [L x (W²)]/2) and plotted +/- SEM. Percent tumor growth inhibition was calculated by calculating the area under the curve (AUC) of each treatment group relative to vehicle control. Student's t-tests were performed on day 21 data to determine significance by p-value.

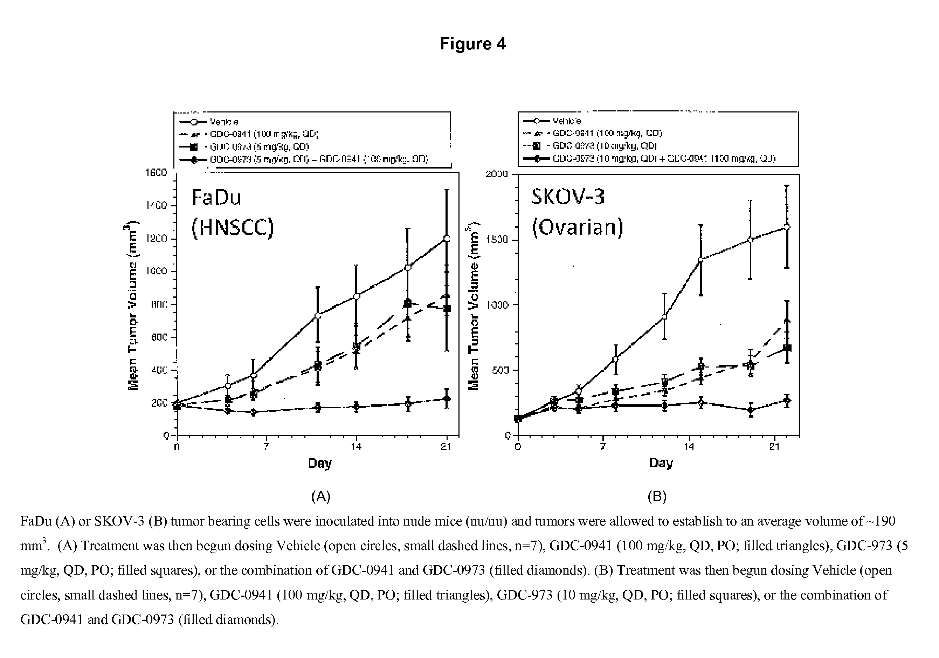

Figure 4 shows combination of GDC-0941 and GDC-0973 in (A) the FaDu (hypopharyngeal squamous cell carcinoma) Xenograft model. (A) Treatment was then begun dosing Vehicle (open circles)), GDC-0941 (100 mg/kg, QD, PO; filled triangles), GDC-973 (5 mg/kg, QD, PO; filled squares), or the combination of GDC-0941 and GDC-0973 (filled diamonds, solid lines, n=7). (B) the SKOV-4 (ovarian) Xenograph model. Treatment was then begun dosing Vehicle (open circles,), GDC-0941 (100 mg/kg, QD, PO; filled triangles), GDC-0973 (10 mg/kg, QD, PO; filled squares), or the combination of GDC-0941 and GDC-0973 (filled diamonds). Caliper measurements of animals' tumors and animal weights were taken every 3-4 days throughout the study and tumor volumes were calculated (TV = [L x (W²)]/2) and plotted +/- SEM. Percent tumor growth inhibition was calculated by calculating the area under the curve (AUC) of each treatment group relative to vehicle control. Student's t-tests were performed on day 21 data to determine significance by p-value.

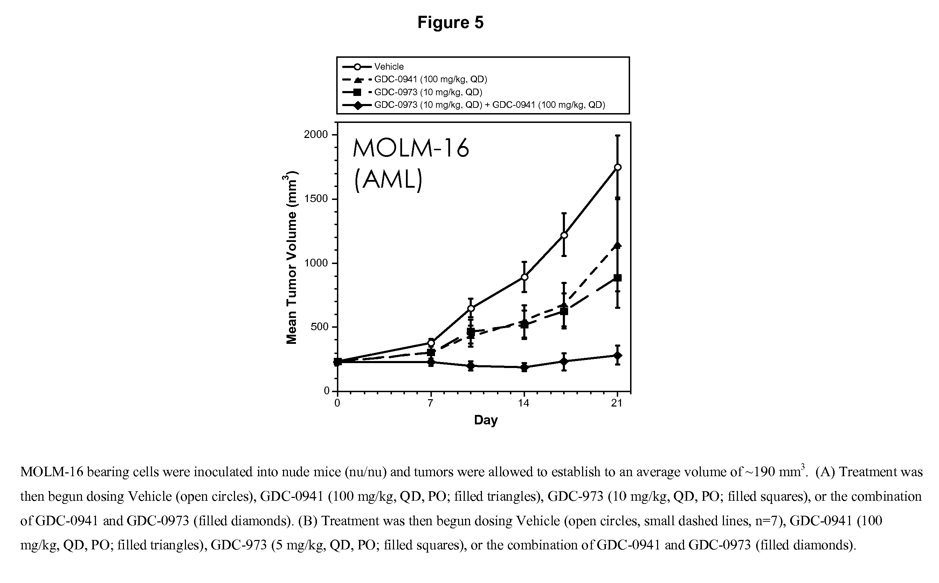

Figure 5 shows combination of GDC-0941 and GDC-0973 in (A) the MOLM-16 (acute myeloid leukemia) Xenograft model. ##A2058 cells (10 x 10⁶ in Hanks Balance Salt Solution (HBSS) + Matrigel) were inoculated into nude mice (nu/nu) and tumors were allowed to establish to an average volume oaf - 190 mm³. (A) Treatment was then begun dosing Vehicle (open circles)), GDC-0941 (100 mg/kg, QD, PO; filled triangles), GDC-973 (10 mg/kg, QD, PO; filled squares), or the combination of GDC-0941 and GDC-0973 (filled diamonds, solid lines, n=7). (B) the MX-1 (triple negative breast) Xenograph model. Treatment was then begun dosing Vehicle (open circles,), GDC-0941 (100 mg/kg, QD, PO; filled triangles), GDC-0973 (5 mg/kg, QD, PO; filled squares), or the combination of GDC-0941 and GDC-0973 (filled diamonds). Caliper measurements of animals' tumors and animal weights were taken every 3-4 days throughout the study and tumor volumes were calculated (TV = [L x (W²)]/2) and plotted +/- SEM. Percent tumor growth inhibition was calculated by calculating the area under the curve (AUC) of each treatment group relative to vehicle control. Student's t-tests were performed on day 21 data to determine significance by p-value.

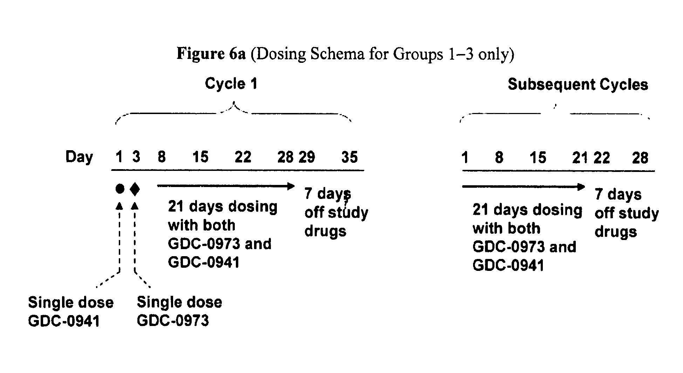

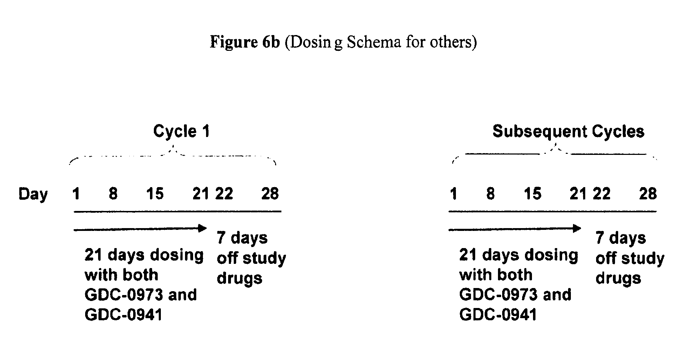

Figures 6a and 6b show dosing schema for GDC-0973 and GDC-0941 combination.

Reference will now be made in detail to certain embodiments of the invention, examples of which are illustrated in the accompanying structures and formulas.

The words "comprise", "comprising", "include", "including" and "includes" when used in this specification and claims are intended to specify the presence of stated features, integers, components, or steps, but they do not preclude the presence or addition of one or more other features, integers, components, steps, or groups thereof.

The terms "treat" and "treating" refer to both therapeutic treatment and prophylactic or preventative measures, wherein the object is to prevent or slow down (lessen) an undesired physiological change or disorder, such as the growth, development or spread of cancer. For purposes of this invention, beneficial or desired clinical results include alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treat" and "treating" can also mean prolonging survival as compared to expected survival if not receiving treatment. Those in need of treatment include those already with the condition or disorder as well as those prone to have the condition or disorder or those in which the condition or disorder is to be prevented.

The term "locally advanced or metastatic solid tumors" includes melanoma, non-small cell lung cancer ("NSCLC"), colorectal cancer, pancreatic cancer, breast cancer and ovarian cancer.

A "metabolite" is a product produced through metabolism in the body of a specified compound or salt thereof. Metabolites of a compound may be identified using routine techniques known in the art and their activities determined using tests such as those described herein. Such products may result e.g. from the oxidation, reduction, hydrolysis, amidation, deamidation, esterification, deesterification, and enzymatic cleavage, of the administered compound. Accordingly, the application includes metabolites of compounds of the invention, including compounds produced by a process comprising contacting a compound of this invention with a mammal for a period of time sufficient to yield a metabolic product thereof.

The phrase "pharmaceutically acceptable salt" as used herein, refers to pharmaceutically acceptable organic or inorganic salts of a compound of the invention. Exemplary salts include sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate "mesylate", ethanesulfonate, benzenesulfonate, *p*-toluenesulfonate, and pamoate (i.e., 1,1'-methylene-bis -(2-hydroxy-3-naphthoate)) salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound of the invention is a base, the desired pharmaceutically acceptable salt may be prepared by any suitable method available in the art, e.g., treatment of the free base with an inorganic acid, such as hydrochloric acid, hydrobromic acid, sulfuric acid, nitric acid, methanesulfonic acid, and phosphoric acid, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid. Acids which are generally considered suitable for the formation of pharmaceutically useful or acceptable salts from basic pharmaceutical compounds are discussed, e.g., by Stahl et al, Camille (eds.) Handbook of Pharmaceutical Salts. Properties, Selection and Use. (2002) Zurich: Wiley-VCH; Berge et al, Journal of Pharmaceutical Sciences (1977) 66(1) 1 19; Gould, International J. of Pharmaceutics (1986) 33 201 217; Anderson et al, The Practice of Medicinal Chemistry (1996), Academic Press, New York; Remington's Pharmaceutical Sciences, 18th ed., (1995) Mack Publishing Co., Easton PA; and in The Orange Book (Food & Drug Administration, Washington, D.C. on their website).

If the compound of the invention is an acid, the desired pharmaceutically acceptable salt may be prepared by any suitable method, e.g., treatment of the free acid with an inorganic or organic base, such as an amine (primary, secondary or tertiary), an alkali metal hydroxide or alkaline earth metal hydroxide. Illustrative examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

The phrase "pharmaceutically acceptable" indicates that the substance or composition must be compatible chemically and/or toxicologically, with the other ingredients comprising a formulation, and/or the mammal being treated therewith.

A "solvate" refers to a physical association or complex of one or more solvent molecules and a compound of the invention. The compounds of the invention may exist in unsolvated as well as solvated forms. Examples of solvents that form solvates include water, isopropanol, ethanol, methanol, DMSO, ethyl acetate, acetic acid, and ethanolamine. The term "hydrate" refers to the complex where the solvent molecule is water. This physical association involves varying degrees of ionic and covalent bonding, including hydrogen bonding. In certain instances the solvate will be capable of isolation, e.g. when one or more solvent molecules are incorporated in the crystal lattice of the crystalline solid. Preparation of solvates is generally known, e.g., Caira et al, J. Pharmaceutical Sci., 93(3), 601 611 (2004). Similar preparations of solvates, hemisolvate, and hydrates are described by van Tonder et al, AAPS PharmSciTech., 5(1), article 12 (2004); and Bingham et al, Chem. Commun., 603 604 (2001). A typical, non-limiting, process involves dissolving the inventive compound in desired amounts of the desired solvent (organic or water or mixtures thereof) at a higher than ambient temperature, and cooling the solution at a rate sufficient to form crystals which are then isolated by standard methods. Analytical techniques such as, e.g. I.R. spectroscopy, show the presence of the solvent (or water) in the crystals as a solvate (or hydrate).

Despite recent advances in human tumor profiling and small and large molecule drug design leading to the discovery of targeted therapeutics that have altered the history of the diseases for which they were initially developed, the overall success rate of targeted agents in oncology is, however, still rather low, which may be partially explained by the heterogeneity of many cancers as well as the complex pathways in which the targets act, which involve multiple redundant pathways and cross-talk among many molecular pathways.

One way to approach this problem is to treat tumors with a combination of targeted agents, such as targeting both the MAPK/ERK pathway and the PI3K/AKT/mTOR pathway. These are pathways that independently and together drive proliferation in many tumors and are usually activated in tumors by a number of genomic events. This approach has a dual benefit: it has the potential to increase the initial tumor response rate in tumors driven by multiple oncogenic events, as well as to decrease the rates of acquired resistance that could occur with either agent alone. This is due to the inhibition of the activating compensatory pathways, which would then prolong the activity of the combination over the activity seen by either agent alone. PI3K-AKT pathway activation has been implicated in several types of cancer (Ward et al., Chem Biol 2003;10:207-13; Cantley, In: The Harvey Lectures, Series 100, 2004-2005. Hoboken: John Wiley and Sons Inc.,2006:103-22). Activating and transforming mutations in the p110α, subunit of PI3K are commonly found in tumors (Bachman et al., Cancer Biol Ther 2004; 3:772-5; Samuels et al., Science 2004;304:554; Karakas et al., Br J Cancer 2006; 94:455-9). In addition, the pathway is activated in numerous types of cancer by receptor tyrosine kinase signaling, RAS mutations, or the loss of the phosphatase PTEN (Cantley, Science 2002;296:1655-7).

Targeting either of these pathways individually can attenuate signaling and has been shown to be efficacious in some animal models (Folkes et al., J Med Chem 2008;51:5522-32; Hoeflich et al., Clin Cancer Res, 2009 15(14):4649-4664)). However, in many tumors, cell proliferation and survival are driven through multiple effector pathways, such as in tumors with concurrent activation of the RAS and PI3K pathways, as is seen frequently in melanoma, lung cancer, and colorectal cancer. In these cases, targeting both of these pathways non-clinically has been shown to be significantly more efficacious than targeting either pathway alone. For example, MEK and PI3K inhibitors have demonstrated improved combination efficacy in KRAS mutant mouse models of lung cancer or breast cancer compared with the single agents (Engelman et al., Nat Med 2008;14:1351-6; Hoeflich et al., Clin Cancer Res, 2009 15(14):4649-4664)). Nonclinical data demonstrating in vitro and in vivo combination efficacy of a MEK inhibitor and a PI3K inhibitor are described herein and in US2009/0098135. Because non-clinical models suggests that inhibition of both the PI3K and MEK pathways results in improved efficacy particularly in RAF and RAS mutant genotypes. Hence, a MEK (such as GDC-0973) and PI3K (such as GDC-0941) inhibitor combination is particularly beneficial in RAS/RAF mutant patients with locally advanced or metastatic solid tumors.

The application relates to methods of treating a patient with locally advanced or metastatic solid tumors with 4-(2-(1H-indazol-4-yl)-6-((4-(methylsulfonyl)piperazin-1-yl)methyl)thieno[3,2-d]-pyrimidin-4-yl)morpholine **(I),** also known as GDC-0941 (US 2008/0076768; WO 2006/046031), which inhibits PI3K, in combination with an inhibitor of MEK described in US2009/0156576.

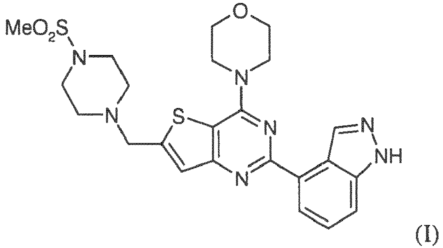

GDC-0941 may be prepared following the methods described in US 2008/0076768, US2008/0207609, US2008/0207611 and US2009/0131429.

The MEK inhibitor useful in combination with GDC-0941, an inhibitor of PI3K, for use in treating patients with locally advanced or metastatic solid tumors, as described s herein, including GDC-0973/XL-518 **(III),** is listed below in Table 1. MEK inhibitors of Table 1 including GDC-0973/XL-518 may be prepared following the methods described in US2009/0156576.

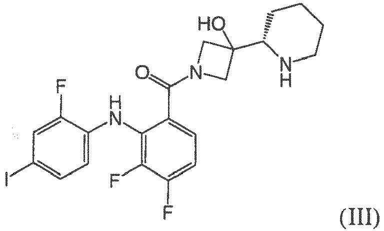

In one embodiment of the application there is provided a method of treating a patient with locally advanced or metastatic solid tumors with 4-(2-(1H-indazol-4-yl)-6-((4-(methylsulfonyl)piperazin-1-yl)methyl)thieno[3,2-d]pyrimidin-4-yl)morpholine, also known as GDC-0941, an inhibitor of PI3K, in combination with an inhibitor of MEK described herein.

In another embodiment of the present application there is provided a method of treating a patient with locally advanced or metastatic solid tumors comprising administering to said patient concurrently GDC-0941 in combination with a MEK inhibitor selected from Table 1, including GDC-0973/XL-518.

In another embodiment of the present application relates to a method of treating a patient with locally advanced or metastatic solid tumors comprising administering to said patient concurrently GDC-0941 **(I)** and GDC-0973/XL-518 (**III**).

In another embodiment of the present application there is provided a method of treating a patient with locally advanced or metastatic solid tumors comprising administering to said patient concurrently GDC-0941 **(I)** in combination with a MEK inhibitor selected from Table 1, including GDC-0973/XL-518 **(III),** wherein said patient is on a 28-day cycle in which said patient is administered with both GDC-0941 and a MEK inhibitor selected from Table 1, including GDC-0973/XL-518 for 21 consecutive days, and no GDC-0941 or a MEK inhibitor selected from Table 1, including GDC-0973/XL-518 for the next 7 consecutive days.

In another embodiment of the present application there is provided a method of treating a patient with locally advanced or metastatic solid tumors comprising administering to said patient concurrently GDC-0941 in combination with a MEK inhibitor selected from Table 1, including GDC-0973/XL-518, wherein said patient is on a 28-day cycle in which said patient is administered with both GDC-0941 and a MEK inhibitor selected from Table 1, including GDC-0973/XL-518 for 14 consecutive days, and no GDC-0941 or a MEK inhibitor selected from Table 1, including GDC-0973/XL-518 for the next 14 consecutive days.

In another embodiment of the present application there is provided a method of treating a patient with locally advanced or metastatic solid tumors comprising administering to said patient concurrently GDC-0941 in combination with a MEK inhibitor selected from Table 1, including GDC-0973/XL-518, wherein said patient is on a 28-day cycle in which said patient is administered with both GDC-0941 and a MEK inhibitor selected from Table 1, including GDC-0973/XL-518 for 21 consecutive days, and no GDC-0941 or a MEK inhibitor selected from Table 1, including GDC-0973/XL-518 for the next 7 consecutive days.

In another embodiment of the present application there is provided a method of treating a patient with locally advanced or metastatic solid tumors comprising administering to said patient concurrently GDC-0941 and GDC-0973/XL-518, wherein said patient is on a 28-day cycle in which said patient is administered with both GDC-0941 and GDC-0973/XL-518 for 14 consecutive days, and no GDC-0941 or GDC-0973/XL-518 for the next 14 consecutive days.

In another embodiment of the present application there is provided a method of treating a patient with locally advanced or metastatic solid tumors comprising administering to said patient concurrently 80mg, 100mg, 130mg or 180mg of GDC-0941 in combination with 20mg, 40mg or 60mg of a MEK inhibitor selected from Table 1, including GDC-0973/XL-518, wherein said patient is on a 28-day cycle in which said patient is administered with both GDC-0941 and MEK inhibitor selected from Table 1, including GDC-0973/XL-518 for 21 consecutive days, and no GDC-0941 or a MEK inhibitor selected from Table 1, including GDC-0973/XL-518 for the next 7 consecutive days.

In another embodiment of the present application there is provided a method of treating a patient with locally advanced or metastatic solid tumors comprising administering to said patient concurrently 80mg, 100mg, 130mg or 180mg of GDC-0941 in combination with 20mg, 40mg or 60mg of a MEK inhibitor selected from Table 1, including GDC-0973/XL-518, wherein said patient is on a 28-day cycle in which said patient is administered with both GDC-094 and a MEK inhibitor selected from Table 1, including GDC-0973/XL-518 for 14 consecutive days, and no GDC-0941 or a MEK inhibitor selected from Table 1, including GDC-0973/XL-518 for the next 14 consecutive days.

In another embodiment of the present application there is provided a method of treating a patient with locally advanced or metastatic solid tumors comprising administering to said patient concurrently 80mg, 100mg, 130mg or 180mg of GDC-0941 and 20mg, 40mg or 60mg of GDC-0973/XL-518, wherein said patient is on a 28-day cycle in which said patient is administered with both GDC-0941 and GDC-0973/XL-518 for 21 consecutive days, and no GDC-0941 or GDC-0973/XL-518 for the next 7 consecutive days.

In another embodiment of the present application there is provided a method of treating a patient with locally advanced or metastatic solid tumors comprising administering to said patient concurrently 80mg, 100mg, 130mg or 180mg of GDC-0941 and 20mg, 40mg or 60mg of GDC-0973/XL-518, wherein said patient is on a 28-day cycle in which said patient is administered with both GDC-0941 and GDC-0973/XL-518 for 14 consecutive days, and no GDC-0941 or GDC-0973/XL-518 for the next 14 consecutive days.

In another embodiment of the present application there is provided methods of treating a patient with RAS/RAF mutant locally advanced or metastatic solid tumors with 4-(2-(1H-indazol-4-yl)-6-((4-(methylsulfonyl)piperazin-1-yl)methyl)thieno[3,2-d]pyrimidin-4-yl)morpholine, also known as GDC-0941, an inhibitor of PI3K, in combination with an inhibitor of MEK described in herein.

In another embodiment of the present application there is provided a method of treating a patient with RAS/RAF mutant locally advanced or metastatic solid tumors comprising administering to said patient concurrently GDC-0941 in combination with a MEK inhibitor selected from Table 1, including GDC-0973/XL-518.

In another embodiment of the present application there is provided a method of treating a patient with RAS/RAF mutant locally advanced or metastatic solid tumors comprising administering to said patient concurrently GDC-0941and GDC-0973/XL-518.

In another embodiment of the present application there is provided a method for the treatment of a patient with a locally advanced or metastatic solid tumor comprising administering a therapeutic combination, as a combined formulation or by alternation, to a mammal wherein the therapeutic combination comprises a therapeutically effective amount of a compound of formula **I,** or a pharmaceutically acceptable salt of **I,** and a therapeutically effective amount of a compound of formula **III,** or a pharmaceutically acceptable salt of **III,** wherein said locally advanced or metastatic solid tumors are subject to abnormal regulation of the RAS/RAF/MEK/ERK pathway.

In another embodiment of the present application there is provided a method for the treatment of a patient with a locally advanced or metastatic solid tumor comprising administering a therapeutic combination, as a combined formulation or by alternation, to a mammal wherein the therapeutic combination comprises a therapeutically effective amount of a compound of formula **I,** or a pharmaceutically acceptable salt of **I,** and a therapeutically effective amount of a compound of formula **III,** or a pharmaceutically acceptable salt of **III,** wherein said locally advanced or metastatic solid tumors express mutations of the RAS or RAF genes.

In another embodiment of the present application there is provided a method for the treatment of a patient with a locally advanced or metastatic solid tumor comprising administering a therapeutic combination, as a combined formulation or by alternation, to a mammal wherein the therapeutic combination comprises a therapeutically effective amount of a compound of formula **I,** or a pharmaceutically acceptable salt of **I,** and a therapeutically effective amount of a compound of formula **III,** or a pharmaceutically acceptable salt of **III,** wherein said locally advanced or metastatic solid tumors are subject to abnormal regulation of the PI3K signaling pathway.

In another embodiment of the present application there is provided a method for the treatment of a patient with a locally advanced or metastatic solid tumor comprising administering a therapeutic combination, as a combined formulation or by alternation, to a mammal wherein the therapeutic combination comprises a therapeutically effective amount of a compound of formula **I,** or a pharmaceutically acceptable salt of **I,** and a therapeutically effective amount of a compound of formula **III,** or a pharmaceutically acceptable salt of **III,** wherein said locally advanced or metastatic solid tumors over-express PI3K or Akt, e.g. wherein said locally advanced or metastatic solid tumors express mutations of the PI3K gene or wherein said locally advanced or metastatic solid tumors exhibit loss of the tumor suppressor phosphatase and tensin homolog (PTEN).

In another embodiment of the present application there is provided a method for the treatment of a patient with a locally advanced or metastatic solid tumor comprising administering a therapeutic combination, as a combined formulation or by alternation, to a mammal wherein the therapeutic combination comprises a therapeutically effective amount of a compound of formula **I,** or a pharmaceutically acceptable salt of **I,** and a therapeutically effective amount of a compound of formula **III,** or a pharmaceutically acceptable salt of **III,** wherein said locally advanced or metastatic solid tumors are selected from the group consisting of pancreatic adenocarcinoma, colorectal adenocarcinoma, non-small cell lung cancer, malignant melanoma, papillary thyroid cancer, breast, ovarian and endometrial cancer.

In another embodiment of the present application there is provided a method of treating a patient with RAS/RAF mutant locally advanced or metastatic solid tumors comprising administering to said patient concurrently GDC-0941 in combination with a MEK inhibitor selected from Table 1, including GDC-0973/XL-518, wherein said patient is on a 28-day cycle in which said patient is administered with both GDC-0941 and a MEK inhibitor selected from Table 1, including GDC-0973/XL-518 for 21 consecutive days, and no GDC-0941 or a MEK inhibitor selected from Table 1, including GDC-0973/XL-518 for the next 7 consecutive days.

In another embodiment of the present application there is provided a method of treating a patient with RAS/RAF mutant locally advanced or metastatic solid tumors comprising administering to said patient concurrently GDC-0941 in combination with a MEK inhibitor selected from Table 1, including GDC-0973/XL-518, wherein said patient is on a 28-day cycle in which said patient is administered with both GDC-0941 and a MEK inhibitor selected from Table 1, including GDC-0973/XL-518 for 14 consecutive days, and no GDC-0941 or a MEK inhibitor selected from Table 1, including GDC-0973/XL-518 for the next 14 consecutive days.

In another embodiment of the present application there is provided a method of treating a patient with RAS/RAF mutant locally advanced or metastatic solid tumors comprising administering to said patient concurrently GDC-0941 in combination with a MEK inhibitor selected from Table 1, including GDC-0973/XL-518, wherein said patient is on a 28-day cycle in which said patient is administered with both GDC-0941 and a MEK inhibitor selected from Table 1, including GDC-0973/XL-518 for 21 consecutive days, and no GDC-0941 or a MEK inhibitor selected from Table 1, including GDC-0973/XL-518 for the next 7 consecutive days.

In another embodiment of the present application there is provided a method of treating a patient with RAS/RAF mutant locally advanced or metastatic solid tumors comprising administering to said patient concurrently GDC-0941 and GDC-0973/XL-518, wherein said patient is on a 28-day cycle in which said patient is administered with both GDC-0941 and GDC-0973/XL-518 for 14 consecutive days, and no GDC-0941 or GDC-0973/XL-518 for the next 14 consecutive days.

In another embodiment of the present application there is provided a method of treating a patient with RAS/RAF mutant locally advanced or metastatic solid tumors comprising administering to said patient concurrently 80mg, 100mg, 130mg or 180mg of GDC-0941 in combination with 20mg, 40mg or 60mg of a MEK inhibitor selected from Table 1, including GDC-0973/XL-518.

In another embodiment of the present application there is provided a method of treating a patient with RAS/RAF mutant locally advanced or metastatic solid tumors comprising administering to said patient concurrently 80mg, 100mg, 130mg or 180mg of GDC-0941 and 20mg, 40mg or 60mg of GDC-0973/XL-518.

In another embodiment of the present application there is provided a method of treating a patient with RAS/RAF mutant locally advanced or metastatic solid tumors comprising administering to said patient concurrently 80mg, 100mg, 130mg or 180mg of GDC-0941 in combination with 20mg, 40mg or 60mg of a MEK inhibitor selected from Table 1, including GDC-0973/XL-518, wherein said patient is on a 28-day cycle in which said patient is administered with both GDC-0941 and MEK inhibitor selected from Table 1, including GDC-0973/XL-518 for 21 consecutive days, and no GDC-0941 or a MEK inhibitor selected from Table 1, including GDC-0973/XL-518 for the next 7 consecutive days.

In another aspect, the application relates to a method of treating a patient with RAS/RAF mutant locally advanced or metastatic solid tumors comprising administering to said patient concurrently 80mg, 100mg, 130mg or 180mg of GDC-0941 in combination with 20mg, 40mg or 60mg of a MEK inhibitor selected from Table 1, including GDC-0973/XL-518, wherein said patient is on a 28-day cycle in which said patient is administered with both GDC-0941 and a MEK inhibitor selected from Table 1, including GDC-0973/XL-518 for 14 consecutive days, and no GDC-0941 or a MEK inhibitor selected from Table 1, including GDC-0973/XL-518 for the next 14 consecutive days.

In another embodiment of the present application there is provided a method of treating a patient with RAS/RAF mutant locally advanced or metastatic solid tumors comprising administering to said patient concurrently 80mg, 100mg, 130mg or 180mg of GDC-0941 and 20mg, 40mg or 60mg of GDC-0973/XL-518, wherein said patient is on a 28-day cycle in which said patient is administered with both GDC-0941 and GDC-0973/XL-518 for 21 consecutive days, and no GDC-0941 or GDC-0973/XL-518 for the next 7 consecutive days.

In another embodiment of the present application there is provided a method of treating a patient with RAS/RAF mutant locally advanced or metastatic solid tumors comprising administering to said patient concurrently 80mg, 100mg, 130mg or 180mg of GDC-0941 and 20mg, 40mg or 60mg of GDC-0973/XL-518, wherein said patient is on a 28-day cycle in which said patient is administered with both GDC-0941 and GDC-0973/XL-518 for 14 consecutive days, and no GDC-0941 or GDC-0973/XL-518 for the next 14 consecutive days.

In another aspect, methods of treatments of the invention include those comprising administering GDC-0941 and a MEK inhibitor selected from Table 1, including GDC-0973/XL-518 in the form of various pharmaceutically acceptable salts and/or pharmaceutical compositions.

The PI3K inhibitor GDC-0941 (I) and MEK inhibitors described herein such as those in Table 1, including GDC-0973/XL-518 (III) include all stereoisomers, geometric isomers, tautomers, metabolites and pharmaceutically acceptable salts thereof.

Pharmaceutical compositions of the invention may further comprise pharmaceutically acceptable carriers, diluents or excipients.

**Table 1**

| All compounds, with the exception of compound 28, are reference compounds. | |
|---|---|
| **Cmpd No.** | **Structure** |
| 1 | 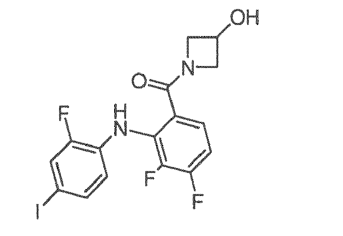 |
| 2 | 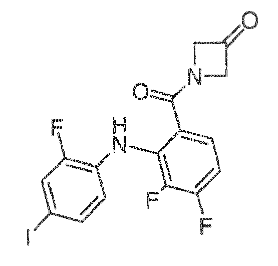 |
| 3 | 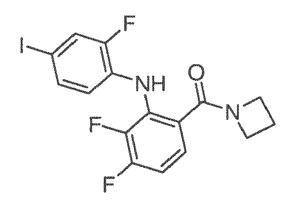 |
| 4 | 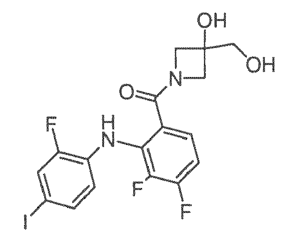 |
| 5 | 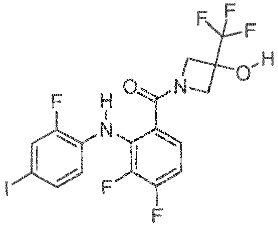 |
| 6 | 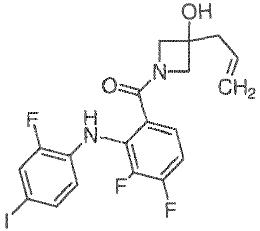 |
| 7 | 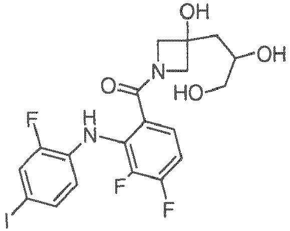 |
| 8 | 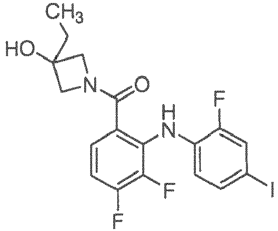 |
| 9 | 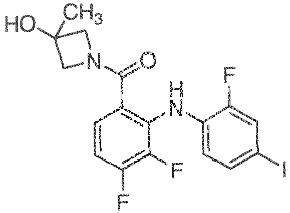 |
| 10 | 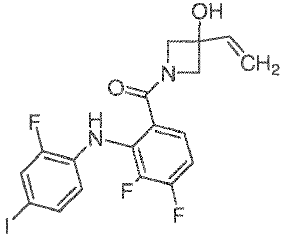 |
| 11 | 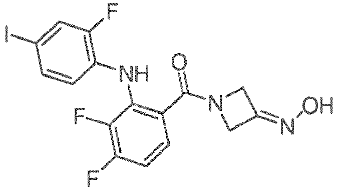 |
| 12 | 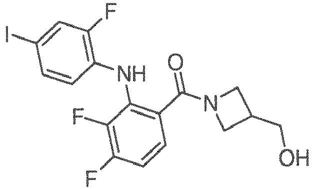 |
| 13 | 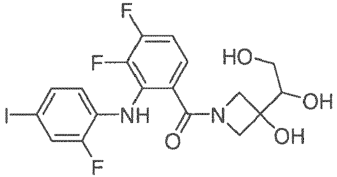 |
| 14 | 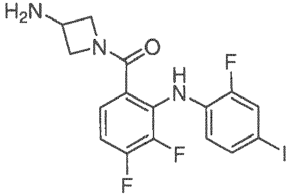 |
| 15 | 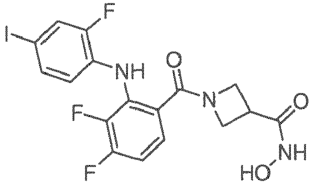 |
| 16 | 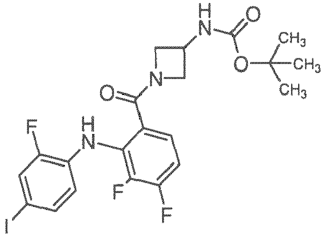 |
| 17 | 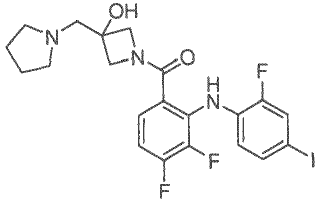 |
| 18 | 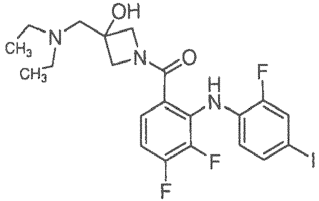 |
| 19 | 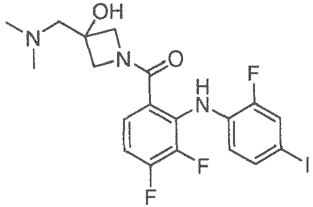 |
| 20 | 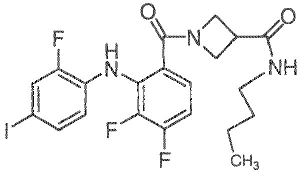 |
| 21 | 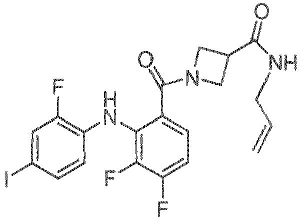 |
| 22 | 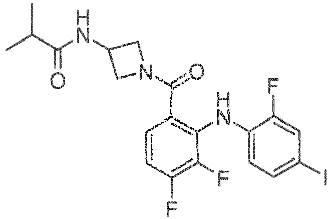 |
| 23 | 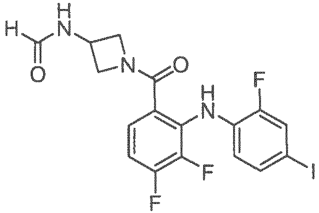 |
| 24 | 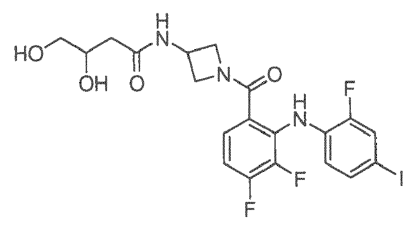 |
| 25 | 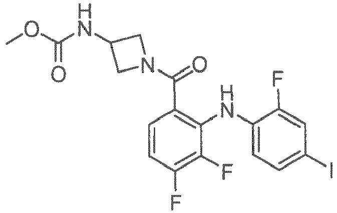 |
| 26 | 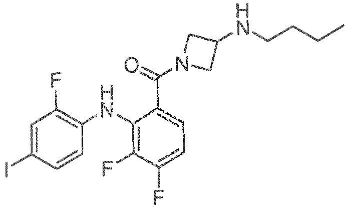 |
| 27 | 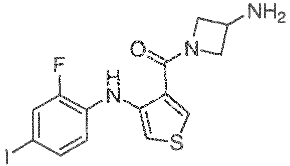 |
| 28 | 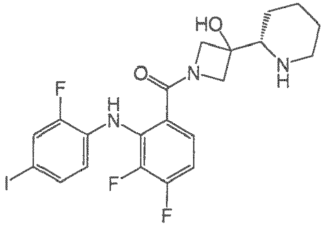 |
| 29 | 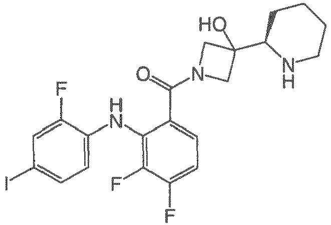 |
| 30 | 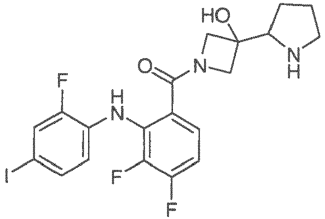 |
| 31 | 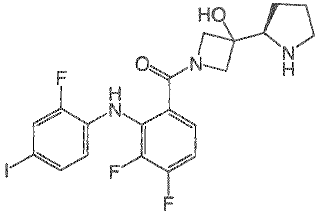 |
| 32 | 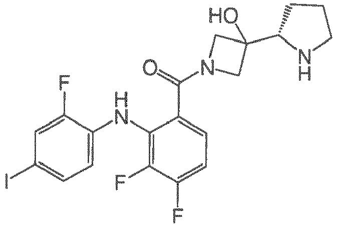 |
| 33 | 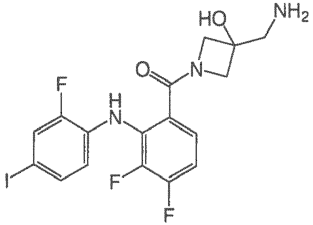 |
| 34 | 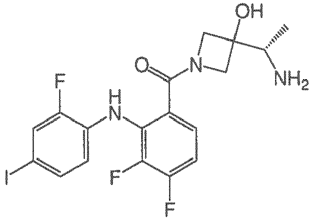 |
| 35 | 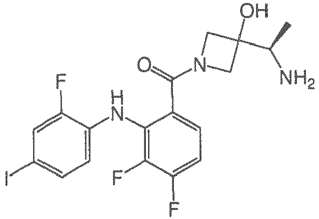 |
| 36 | 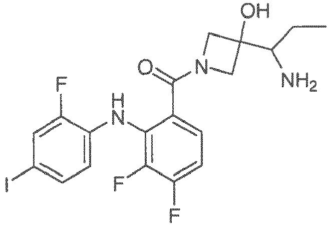 |
| 37 | 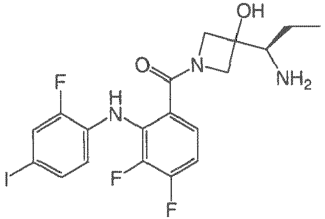 |
| 38 | 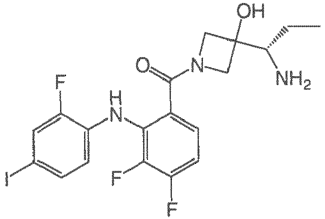 |
| 39 | 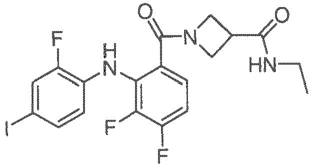 |
| 40 | 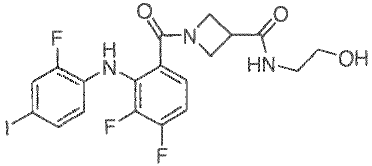 |
| 41 | 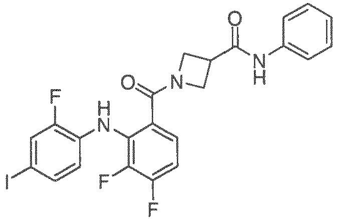 |
| 42 | 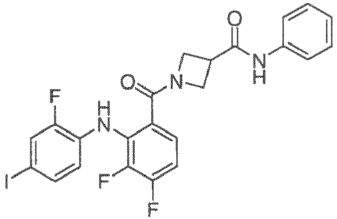 |
| 43 | 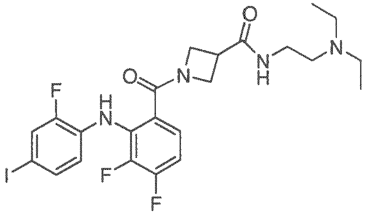 |
| 44 | 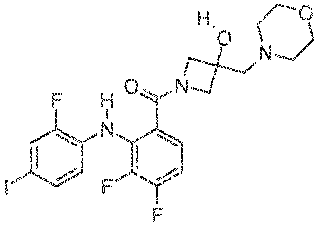 |
| 45 | 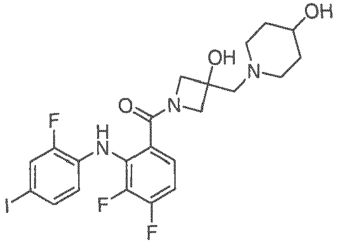 |
| 46 | 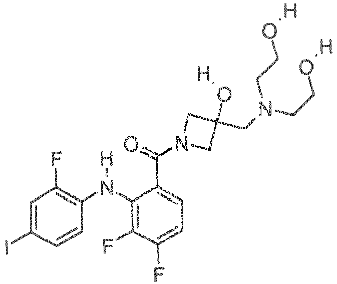 |
| 47 | 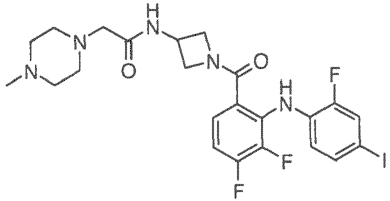 |
| 48 | 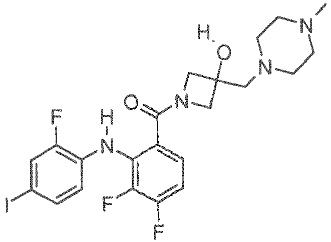 |
| 49 | 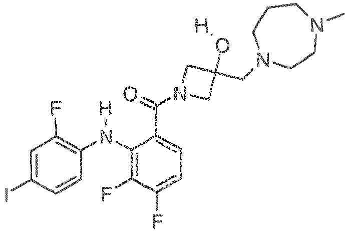 |
| 50 | 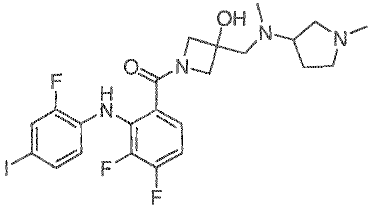 |
| 51 | 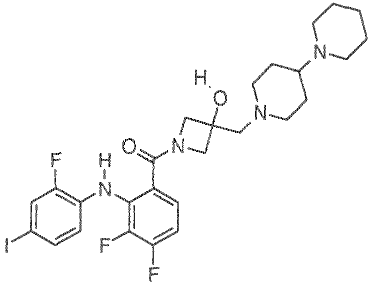 |
| 52 | 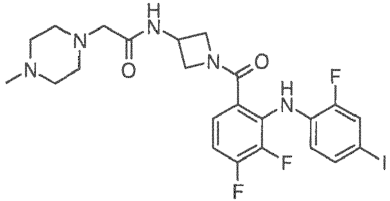 |
| 53 | 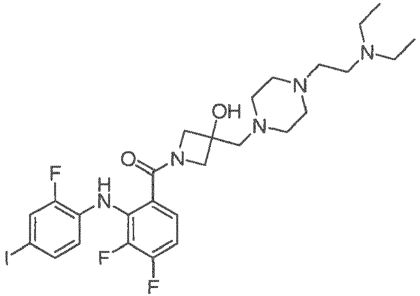 |
| 54 | 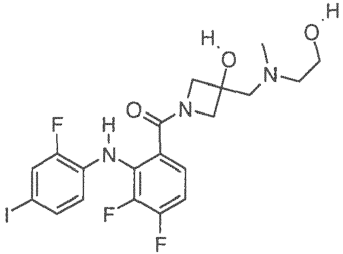 |
| 55 | 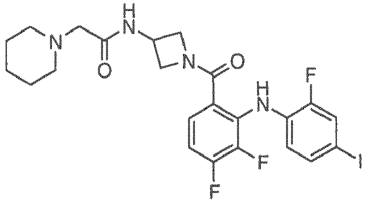 |
| 56 | 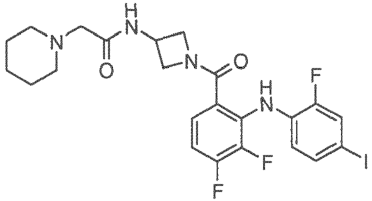 |
| 57 | 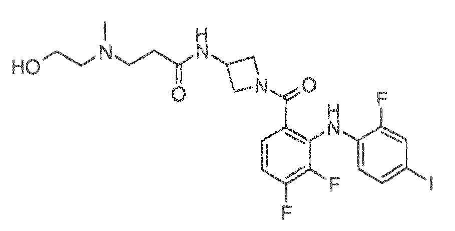 |
| 58 | 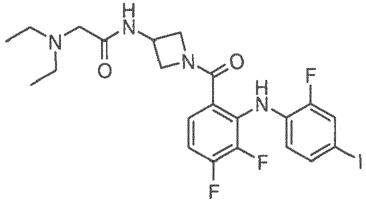 |
| 59 | 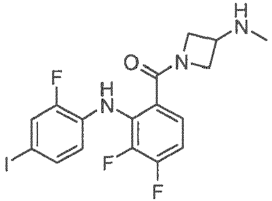 |
| 60 | 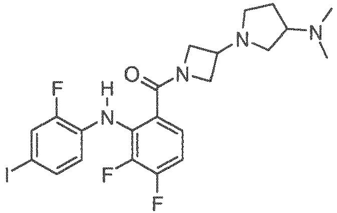 |
| 61 | 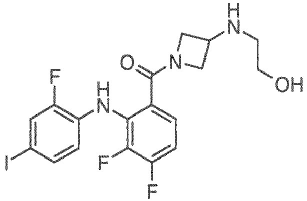 |
| 62 | 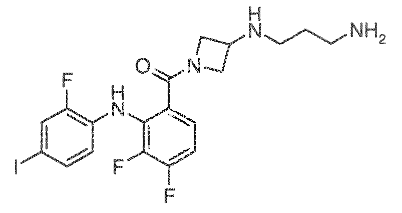 |
| 63 | 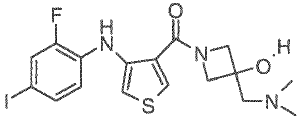 |
| 64 | 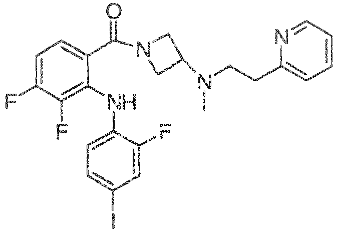 |
| 65 | 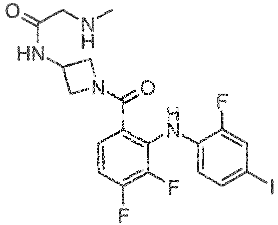 |
| 66 | 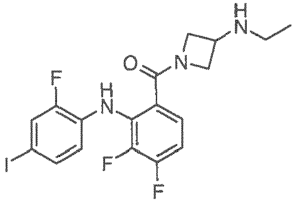 |
| 67 | 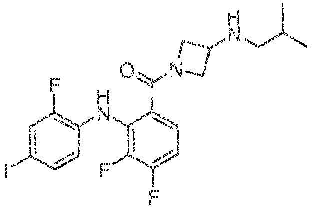 |
| 68 | 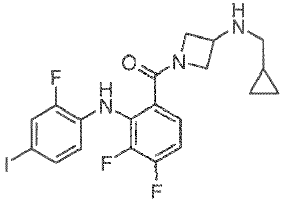 |
| 69 | 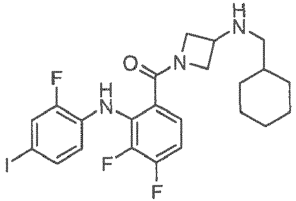 |
| 70 | 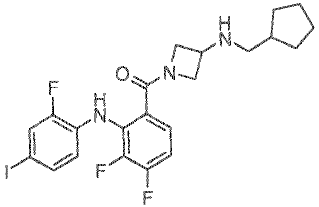 |
| 71 | 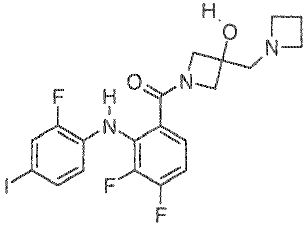 |
| 72 | 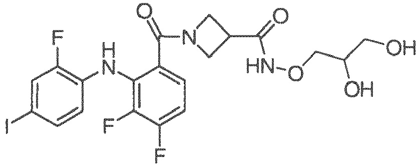 |
| 73 | 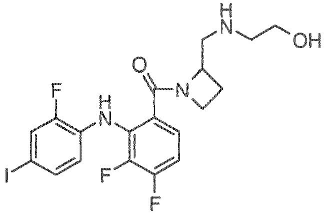 |
| 74 | 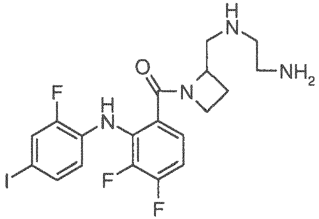 |
| 75 | 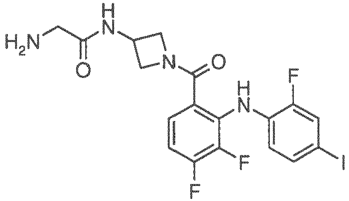 |
| 76 | 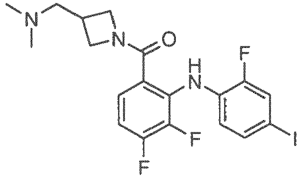 |
| 77 | 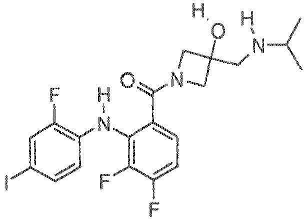 |
| 78 | 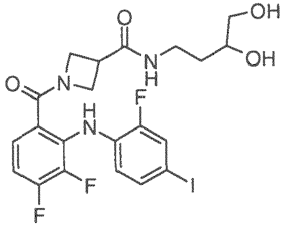 |
| 79 | 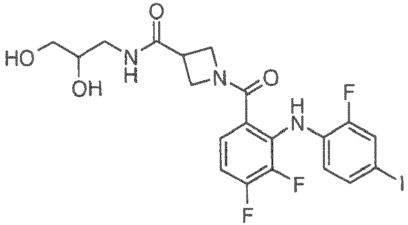 |
| 80 | 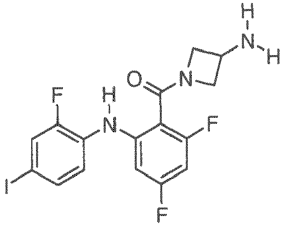 |
| 81 | 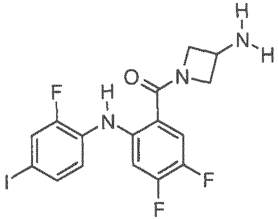 |
| 82 | 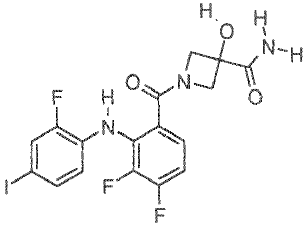 |
| 83 | 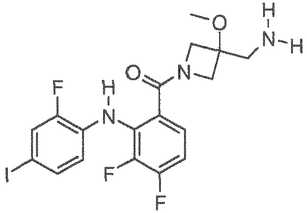 |
| 84 | 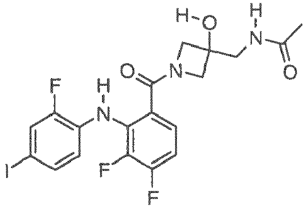 |
| 85 | 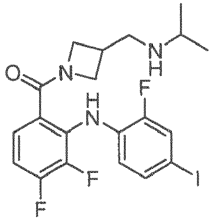 |
| 86 | 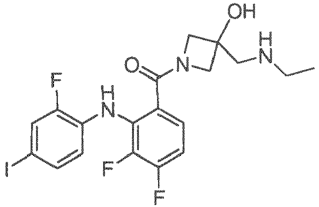 |
| 87 | 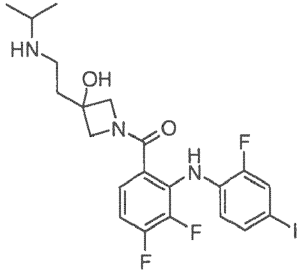 |
| 88 | 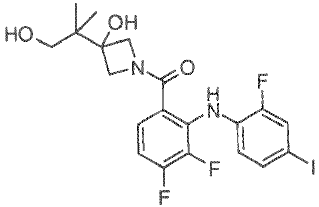 |
| 89 | 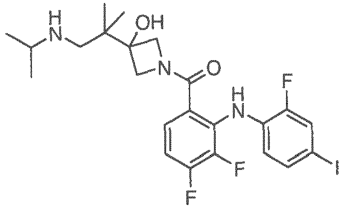 |
| 90 | 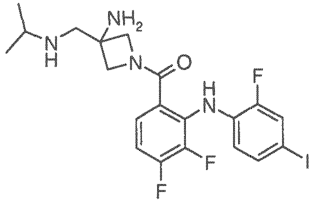 |
| 91 | 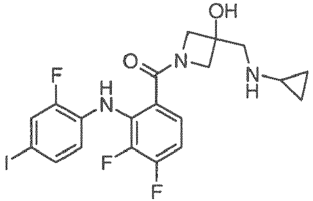 |
| 92 | 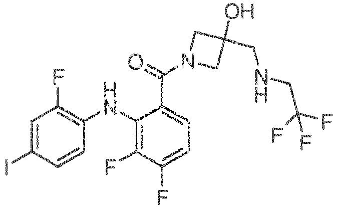 |
| 93 | 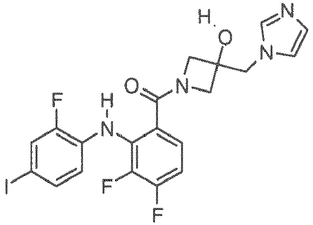 |
| 94 | 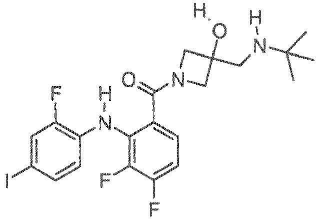 |
| 95 | 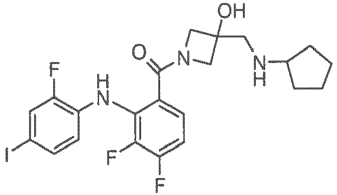 |
| 96 | 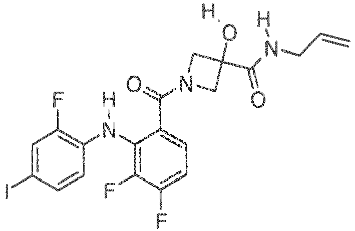 |
| 97 | 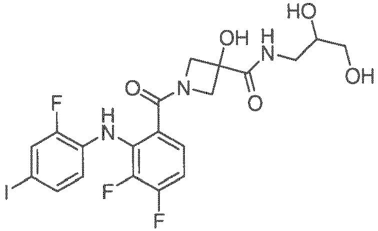 |
| 98 | 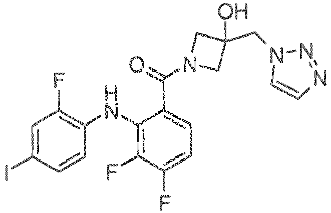 |
| 99 | 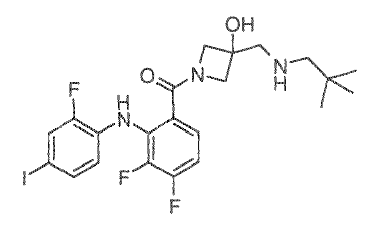 |
| 100 | 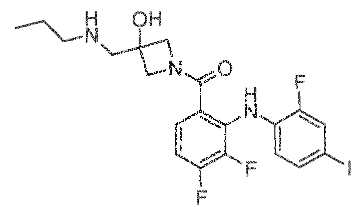 |
| 101 | 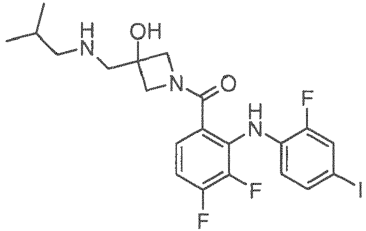 |
| 102 | 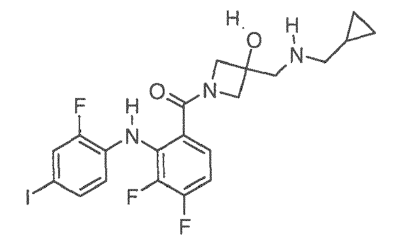 |
| 103 | 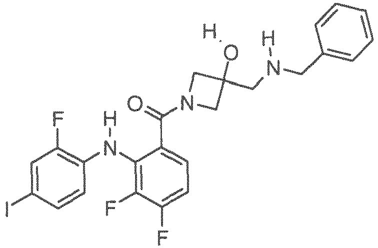 |
| 104 | 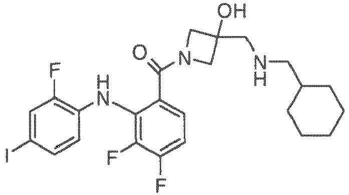 |
| 105 | 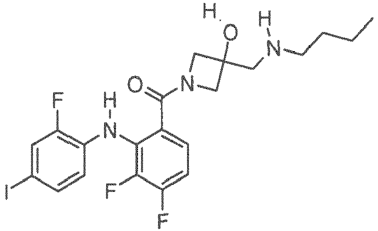 |
| 106 | 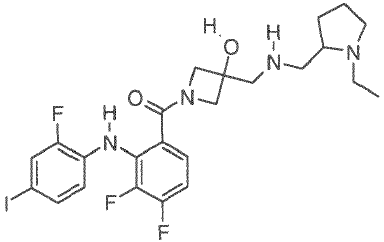 |
| 107 | 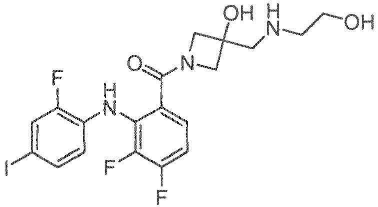 |
| 108 | 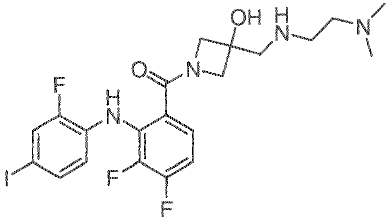 |
| 109 | 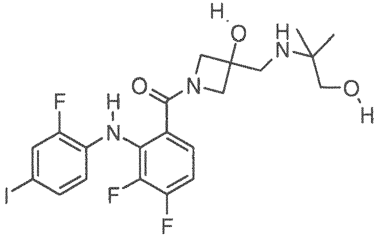 |
| 110 | 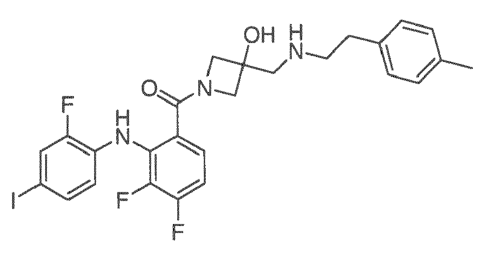 |
| 111 | 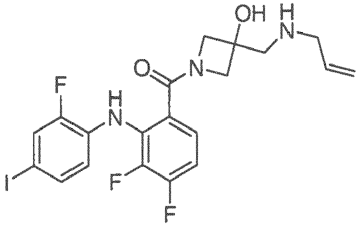 |
| 112 | 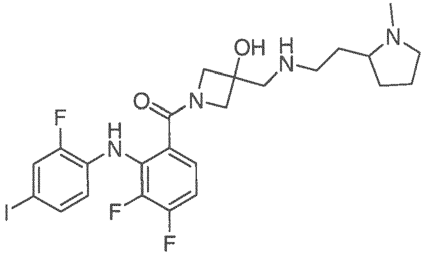 |
| 113 | 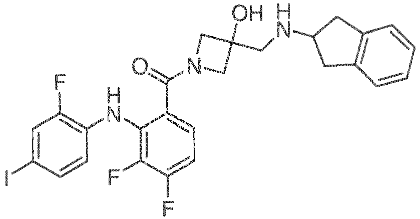 |
| 114 | 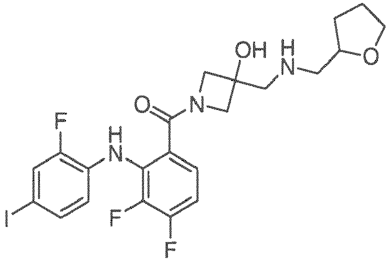 |
| 115 | 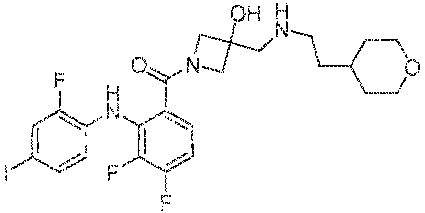 |
| 116 | 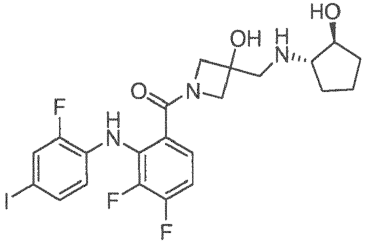 |
| 117 | 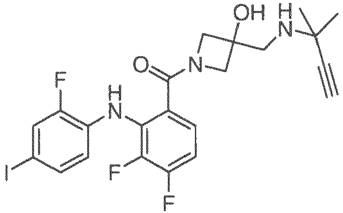 |
| 118 | 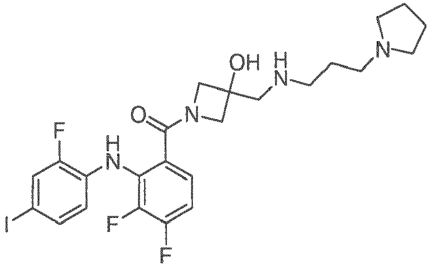 |
| 119 | 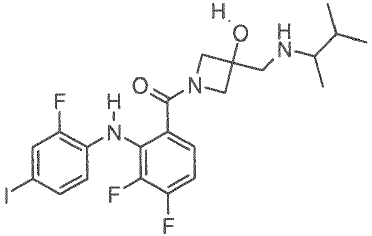 |
| 120 | 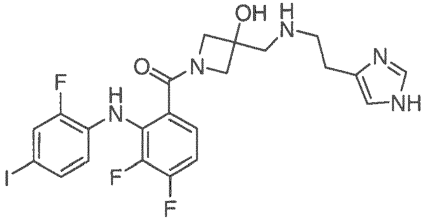 |
| 121 | 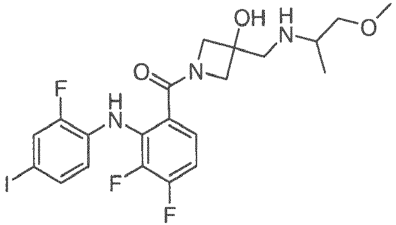 |
| 122 | 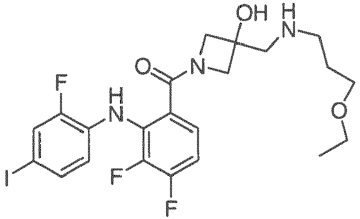 |
| 123 | 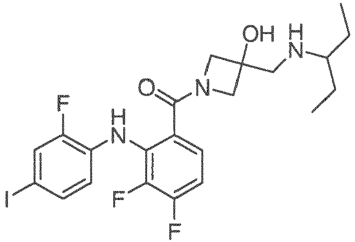 |
| 124 | 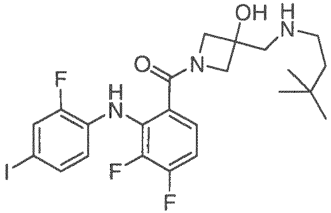 |
| 125 | 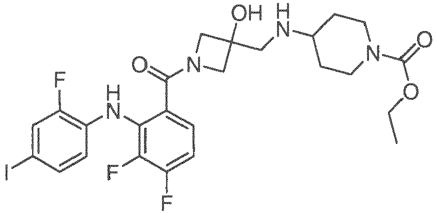 |
| 126 | 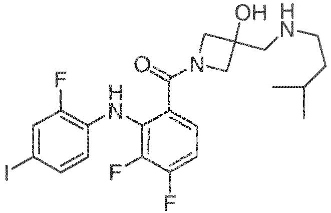 |
| 127 | 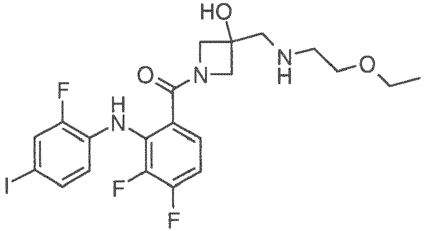 |
| 128 | 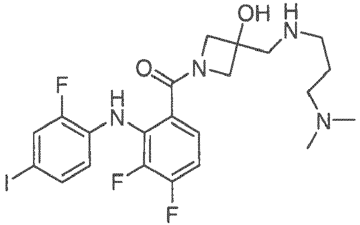 |
| 129 | 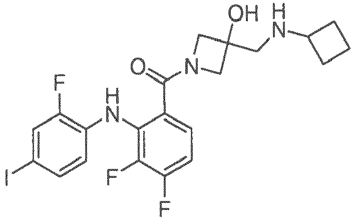 |
| 130 | 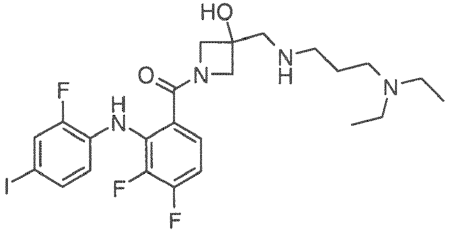 |
| 131 | 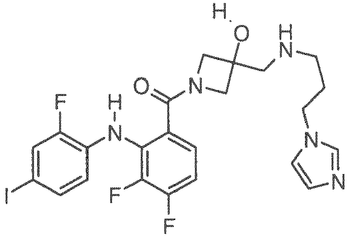 |
| 132 | 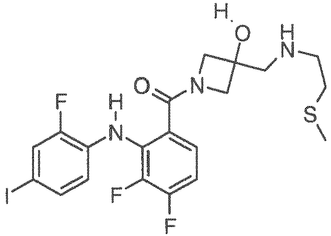 |
| 133 | 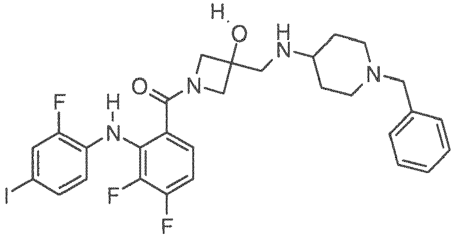 |
| 134 | 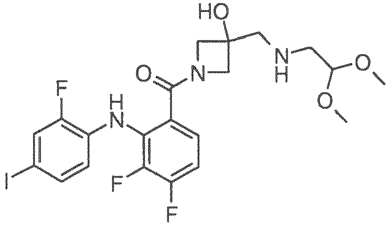 |
| 135 | 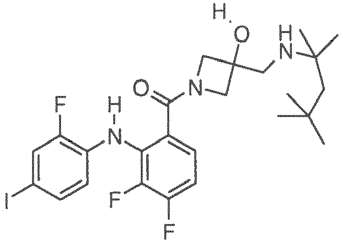 |
| 136 | 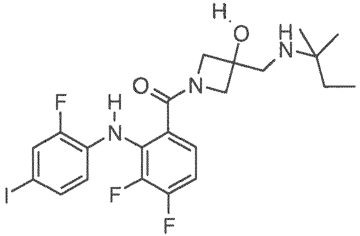 |
| 137 | 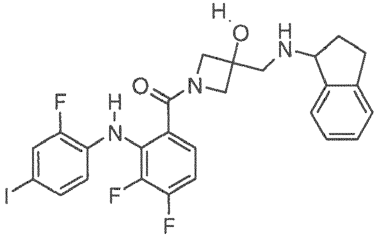 |
| 138 | 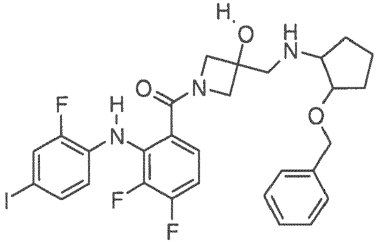 |
| 139 | 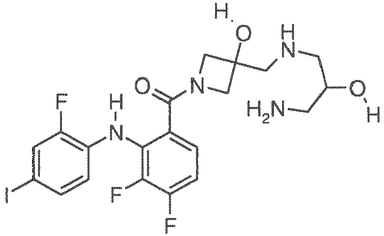 |
| 140 | 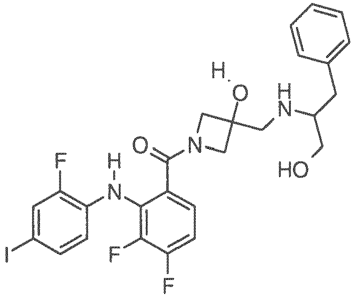 |
| 141 | 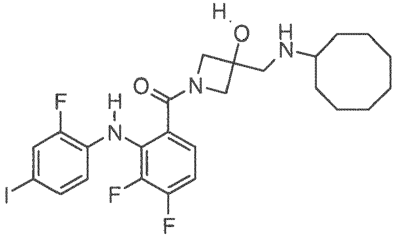 |
| 142 | 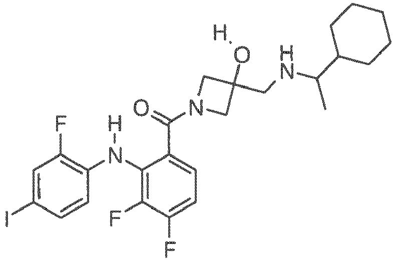 |
| 143 | 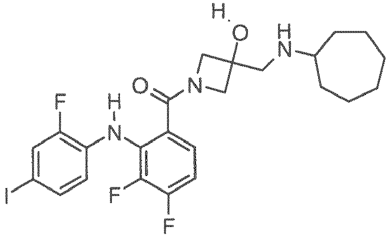 |
| 144 | 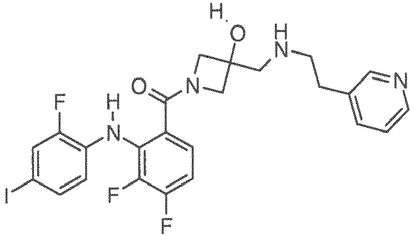 |
| 145 | 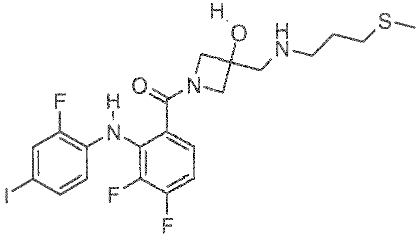 |
| 146 | 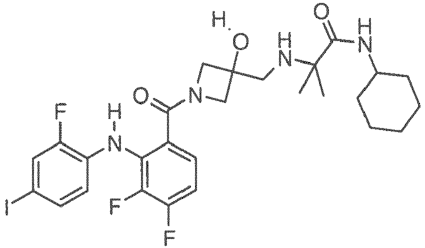 |
| 147 | 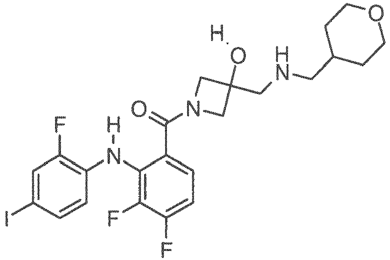 |
| 148 | 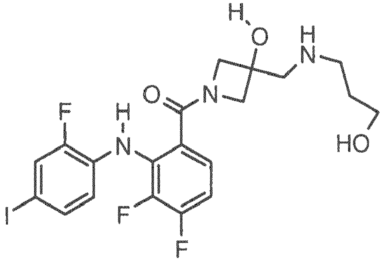 |
| 149 | 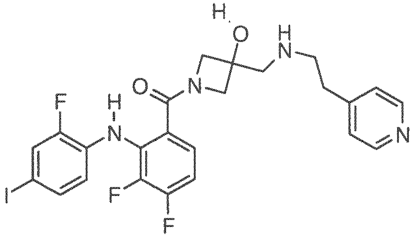 |
| 150 | 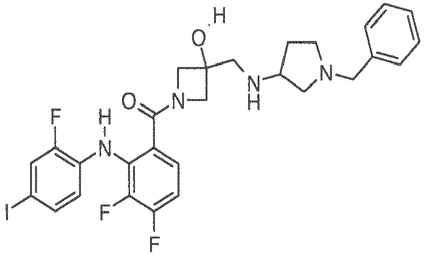 |
| 151 | 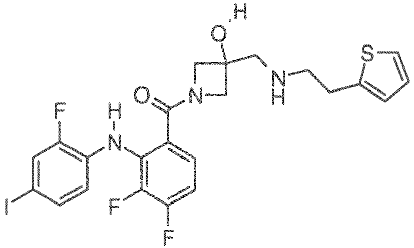 |
| 152 | 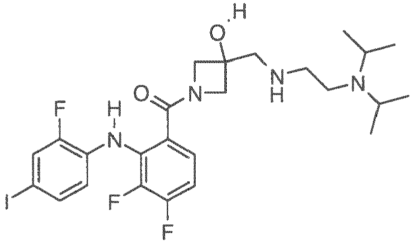 |
| 153 | 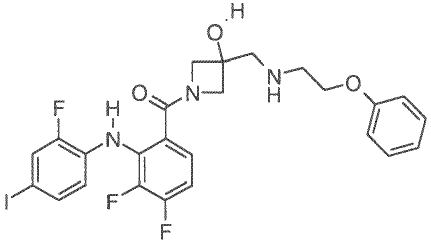 |
| 154 | 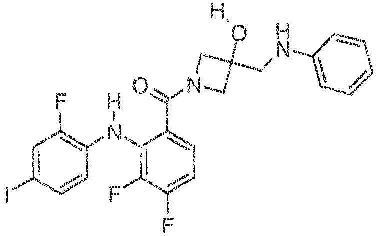 |
| 155 | 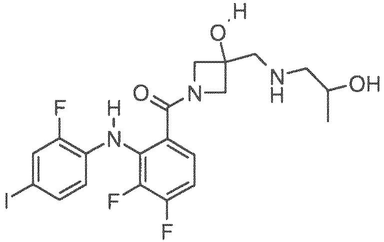 |
| 156 | 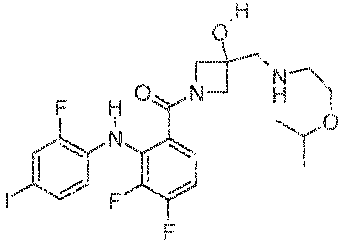 |
| 157 | 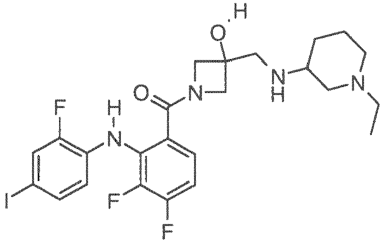 |
| 158 | 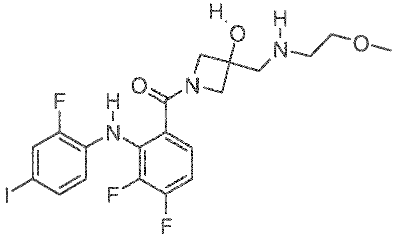 |
| 159 | 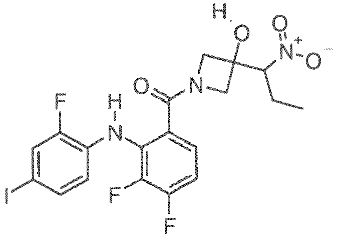 |
| 160 | 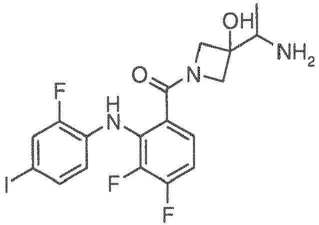 |
| 161 | 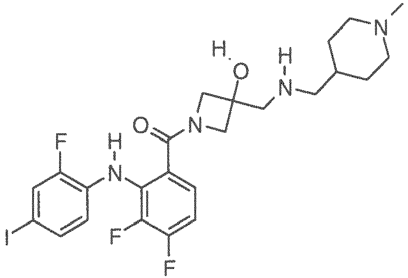 |
| 162 | 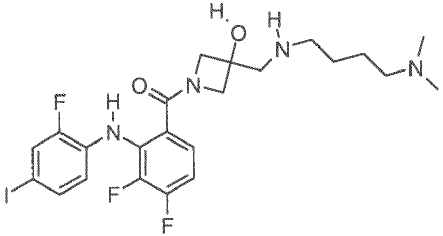 |
| 163 | 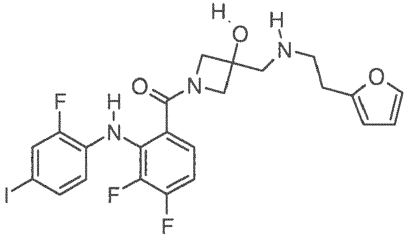 |
| 164 | 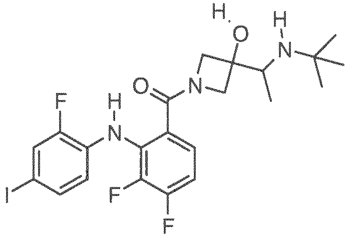 |
| 165 | 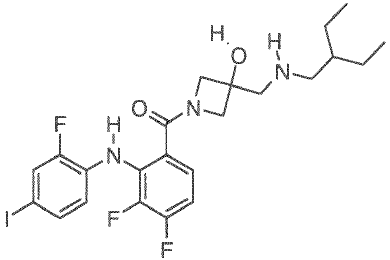 |
| 166 | 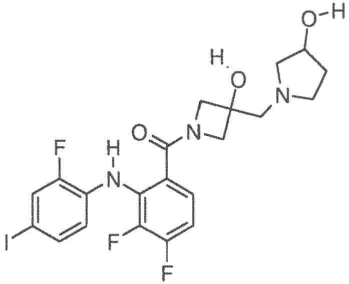 |
| 167 | 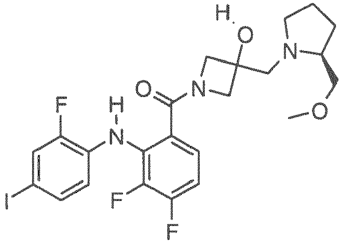 |
| 168 | 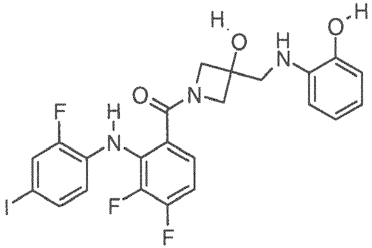 |
| 169 | 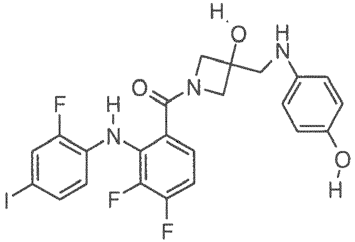 |
| 170 | 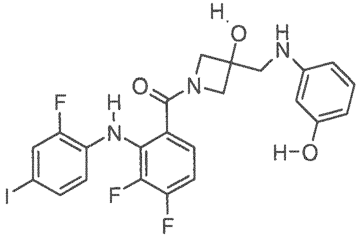 |
| 171 | 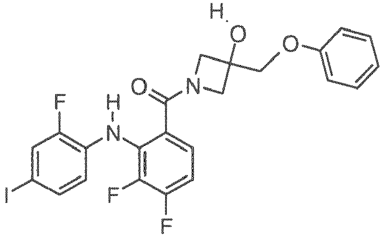 |
| 172 | 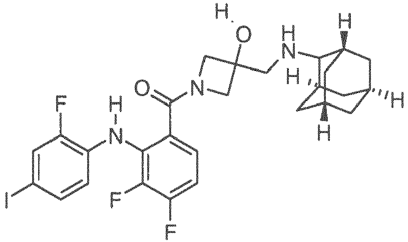 |
| 173 | 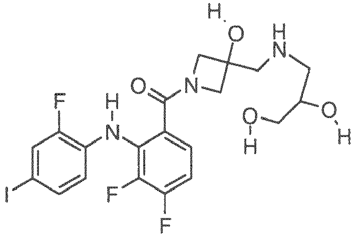 |
| 174 | 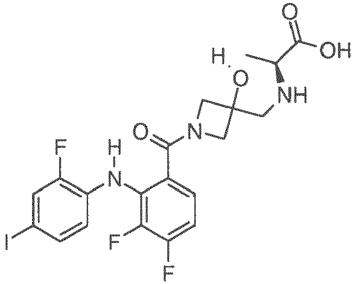 |
| 175 | 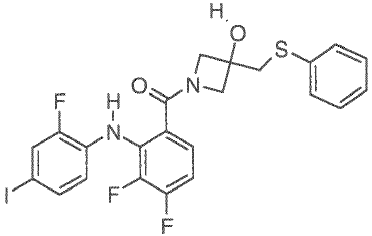 |
| 176 | 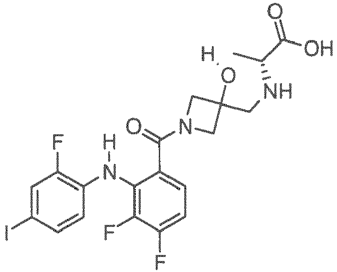 |
| 177 | 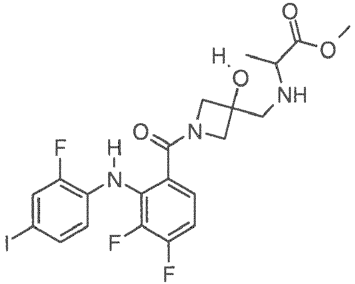 |
| 178 | 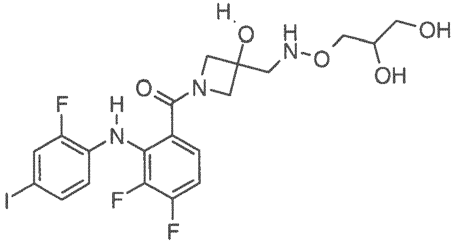 |
| 179 | 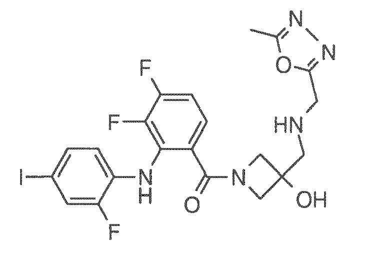 |
| 180 | 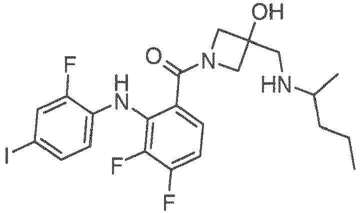 |
| 181 | 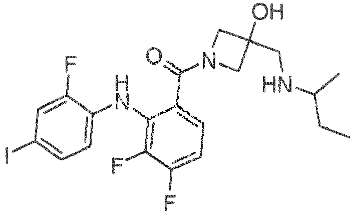 |
| 182 | 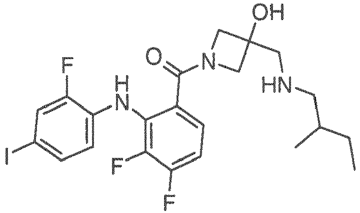 |
| 183 | 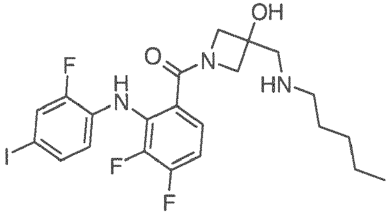 |
| 184 | 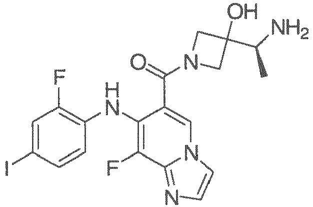 |
| 185 | 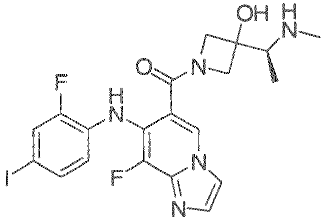 |
| 186 | 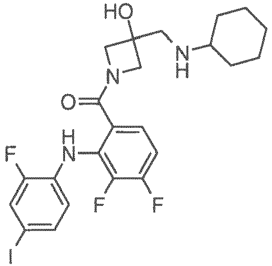 |
| 187 | 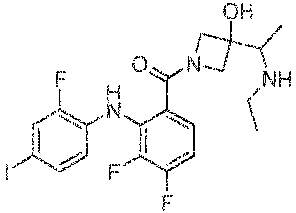 |
| 188 | 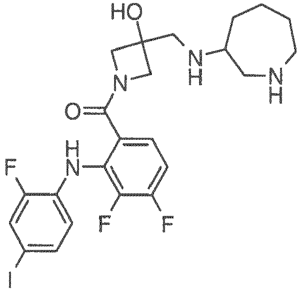 |
| 189 | 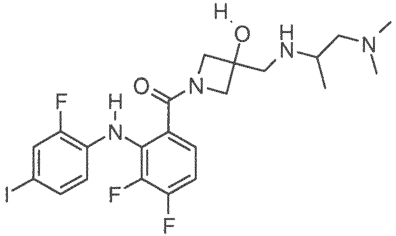 |
| 190 | 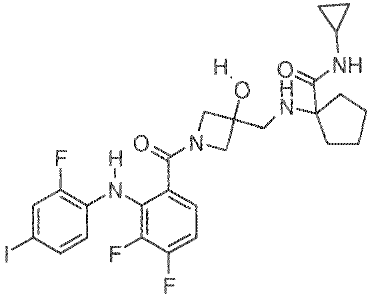 |
| 191 | 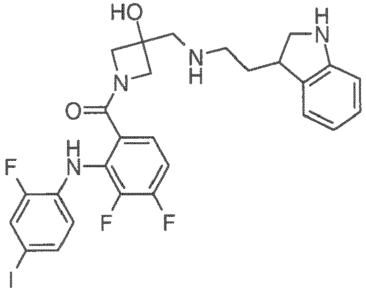 |
| 192 | 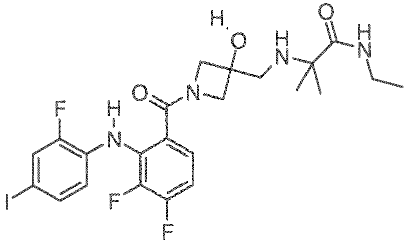 |
| 193 | 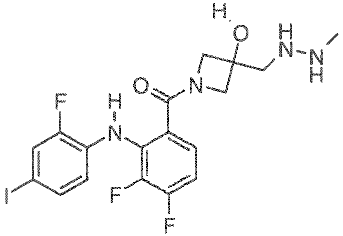 |
| 194 | 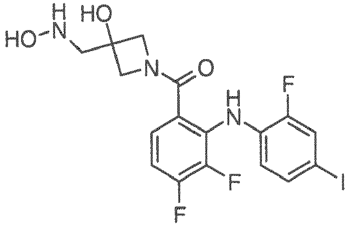 |
| 195 | 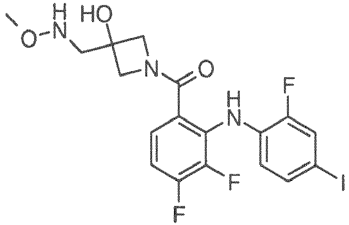 |
| 196 | 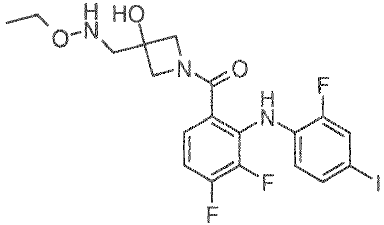 |
| 197 | 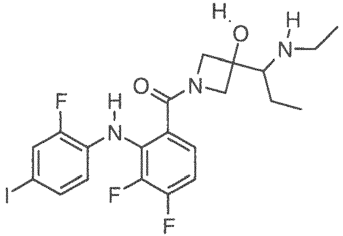 |
| 198 | 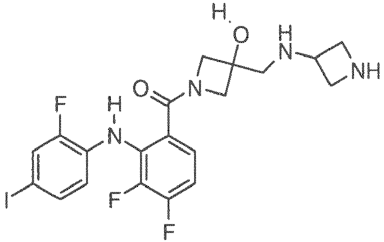 |
| 199 | 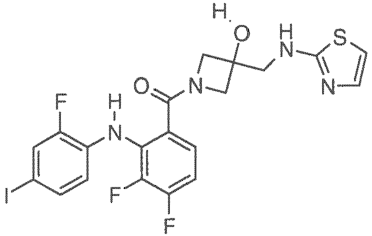 |
| 200 | 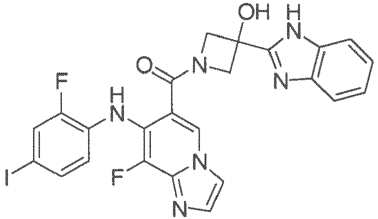 |
| 201 | 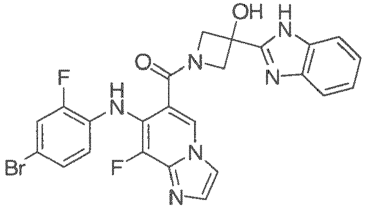 |
| 202 | 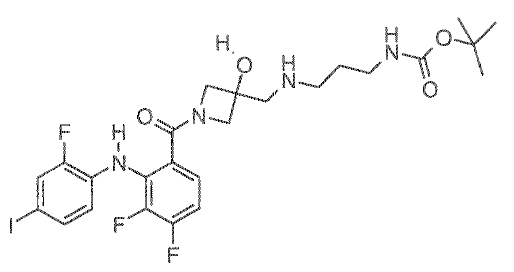 |
| 203 | 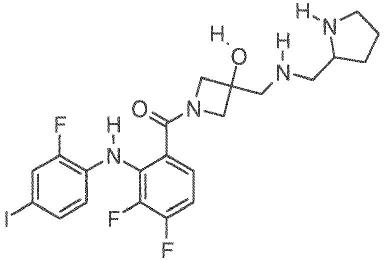 |
| 204 | 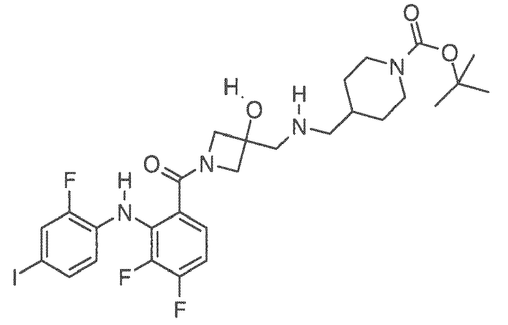 |
| 205 | 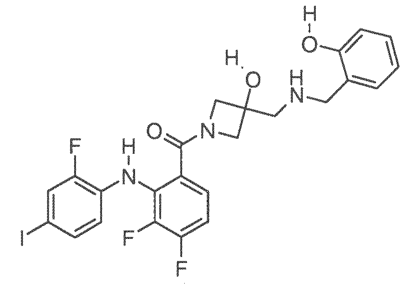 |
| 206 | 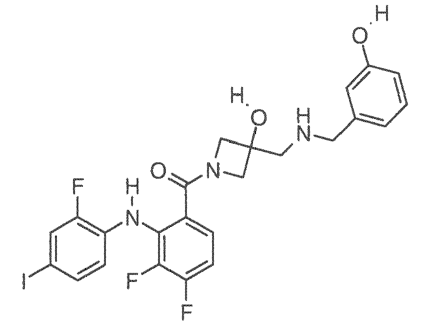 |
| 207 | 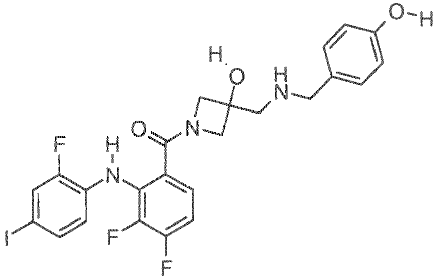 |
| 208 | 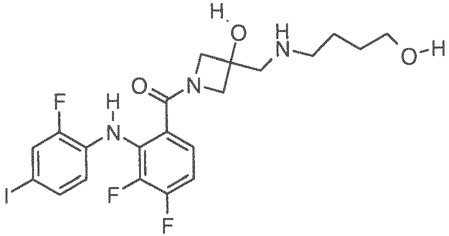 |
| 209 | 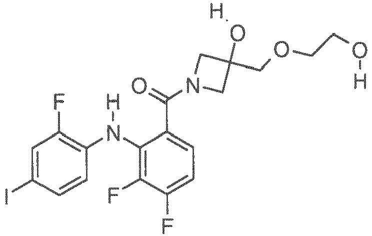 |
| 210 | 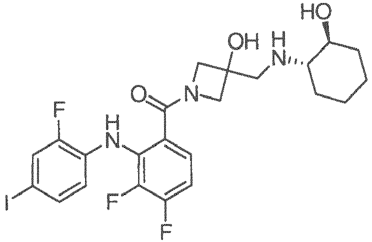 |
| 211 | 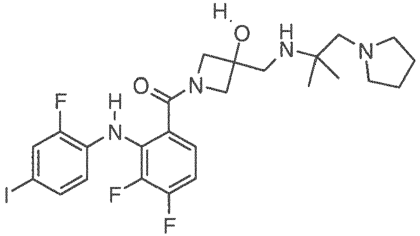 |
| 212 | 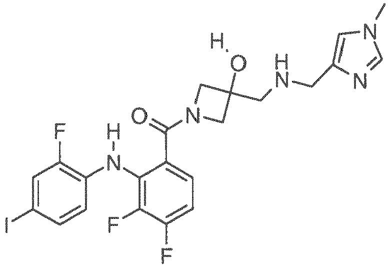 |
| 213 | 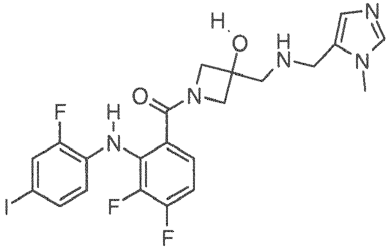 |
| 214 | 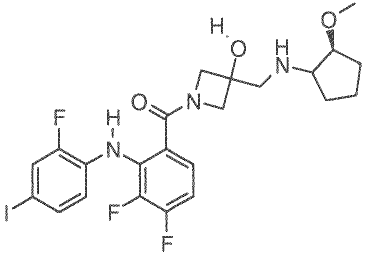 |
| 215 | 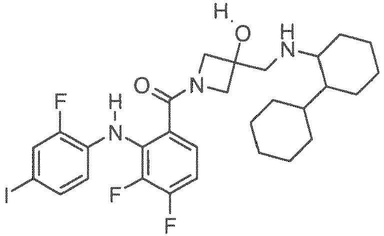 |
| 216 | 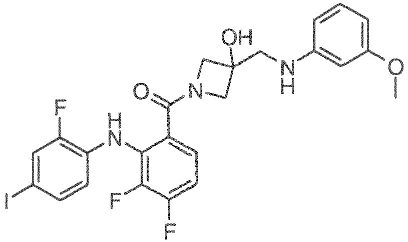 |
| 217 | 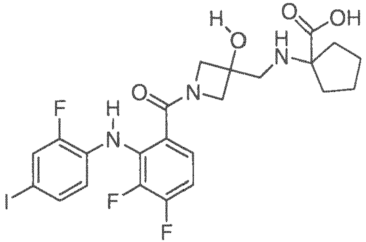 |
| 218 | 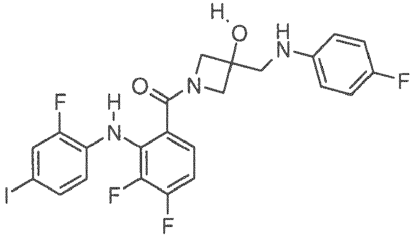 |
| 219 | 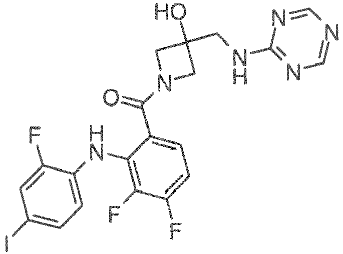 |
| 220 | 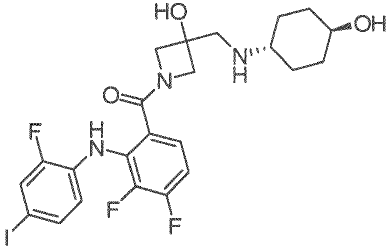 |
| 221 | 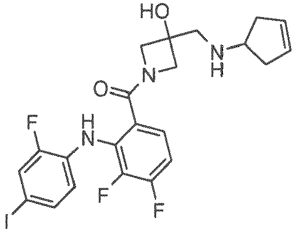 |
| 222 | 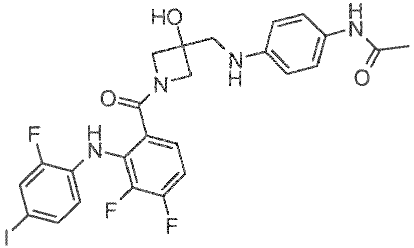 |
| 223 | 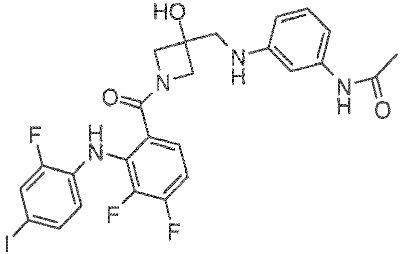 |
| 224 | 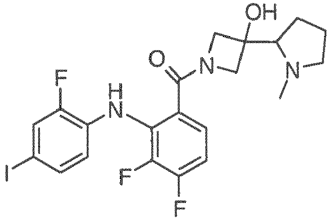 |
| 225 | 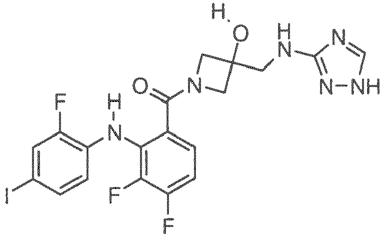 |
| 226 | 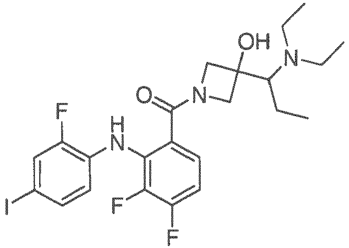 |
| 227 | 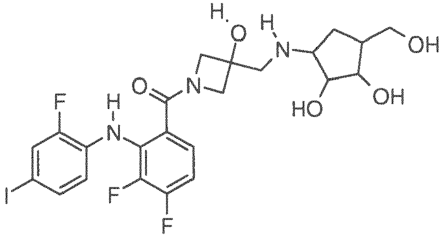 |
| 228 | 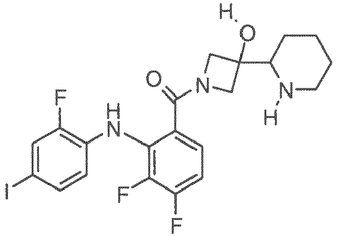 |
| 229 | 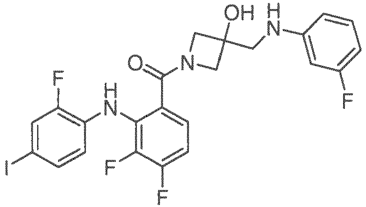 |
| 230 | 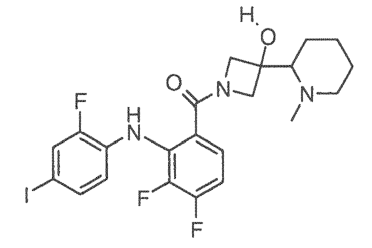 |
| 231 | 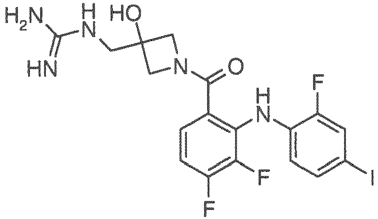 |
| 232 | 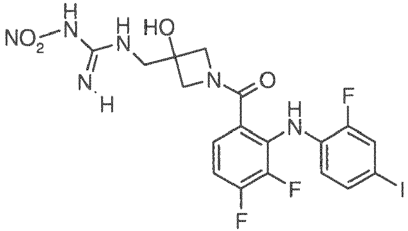 |
| 233 | 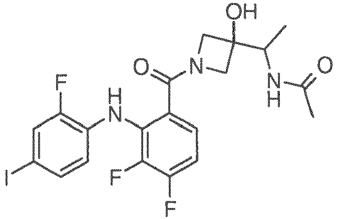 |
| 234 | 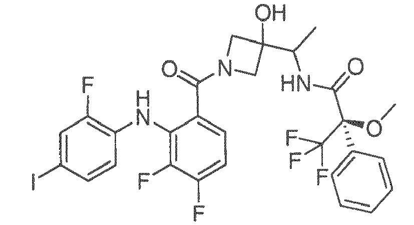 |
| 235 | 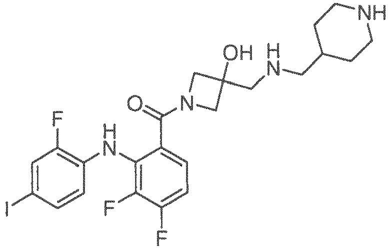 |
| 236 | 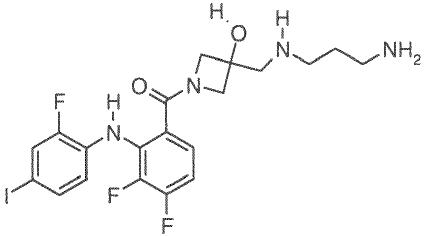 |
| 237 | 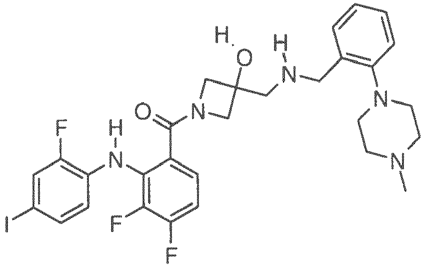 |
| 238 | 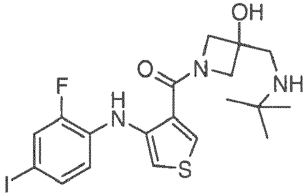 |
| 239 | 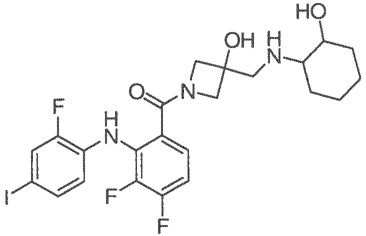 |
| 240 | 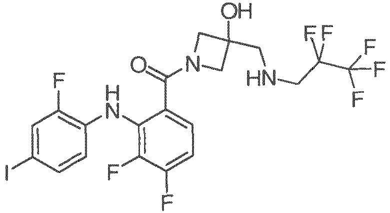 |
| 241 | 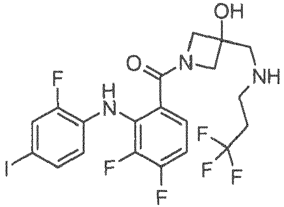 |
| 242 | 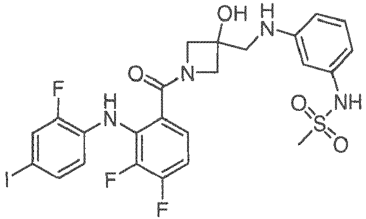 |
| 243 | 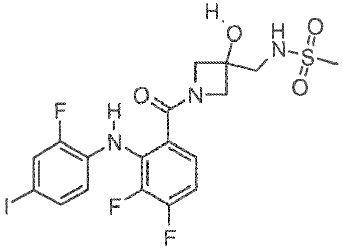 |
| 244 | 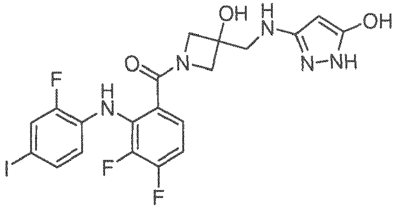 |
| 245 | 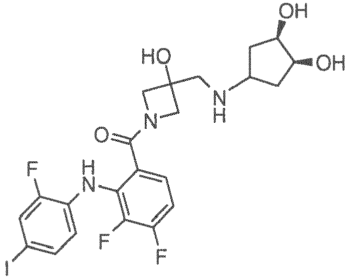 |
| 246 | 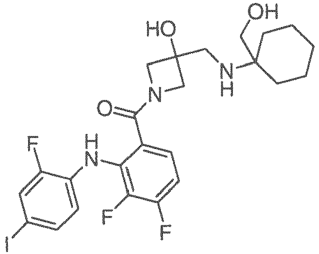 |
| 247 | 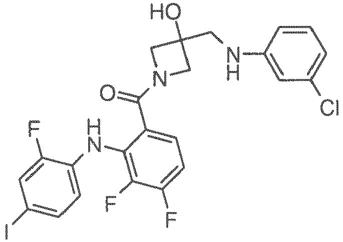 |
| 248 | 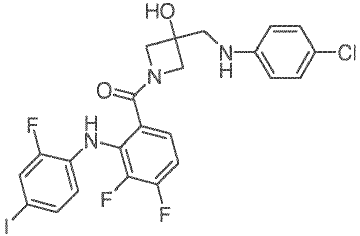 |
| 249 | 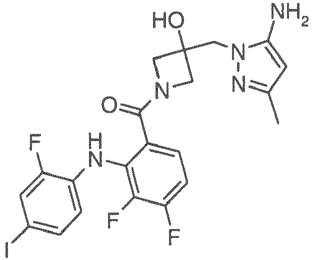 |
| 250 | 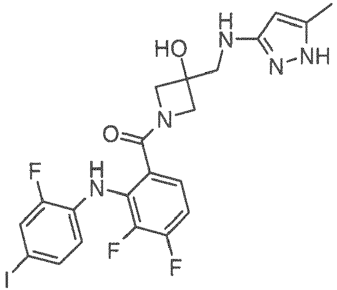 |
| 251 | 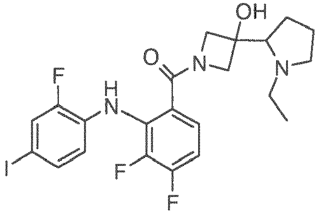 |
| 252 | 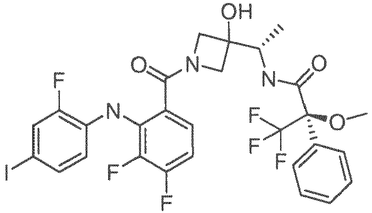 |
| 253 | 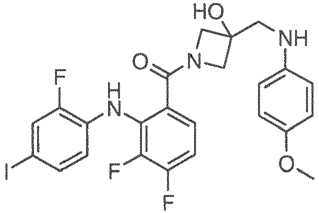 |
| 254 | 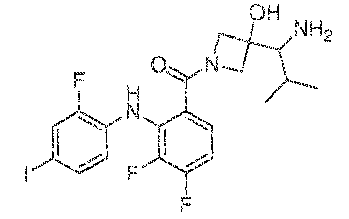 |
| 255 | 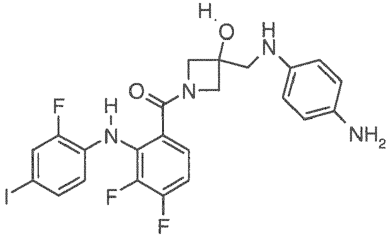 |
| 256 | 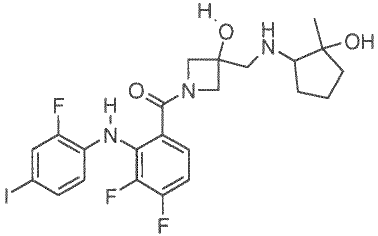 |
| 257 | 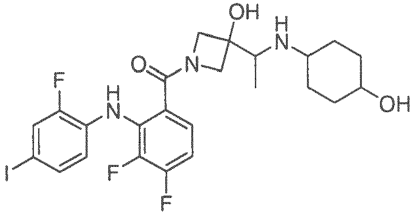 |
| 258 | 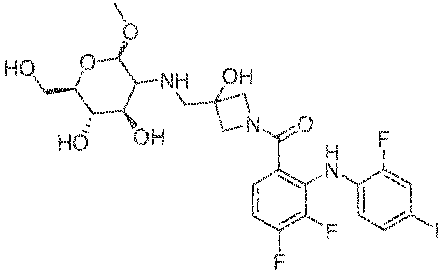 |
| 259 | 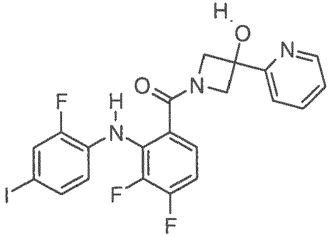 |
| 260 | 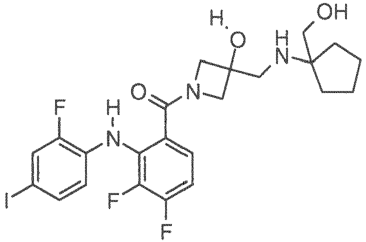 |
| 261 | 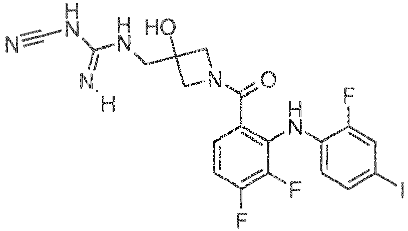 |
| 262 | 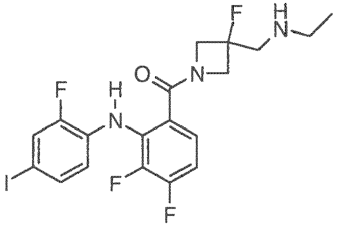 |
| 263 | 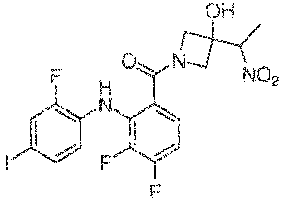 |
| 264 | 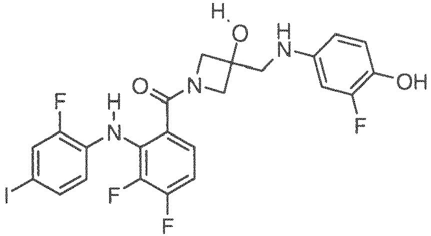 |
| 265 | 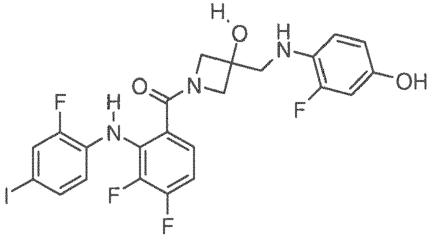 |
| 266 | 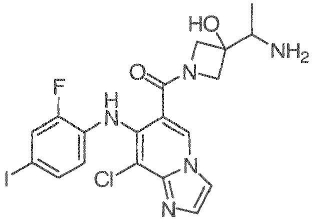 |
| 267 | 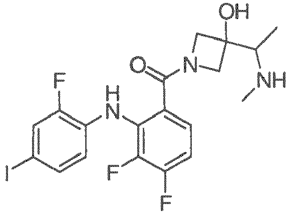 |
| 268 | 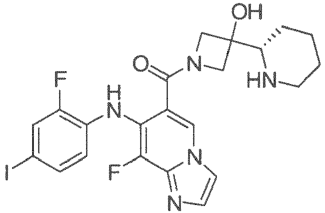 |
| 269 | 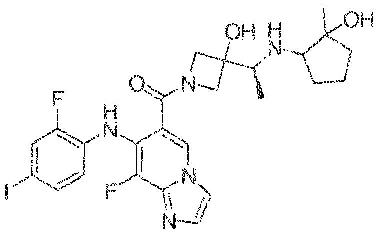 |
| 270 | 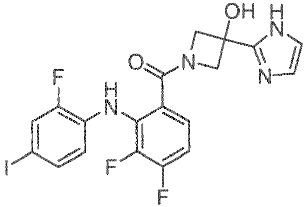 |
| 271 | 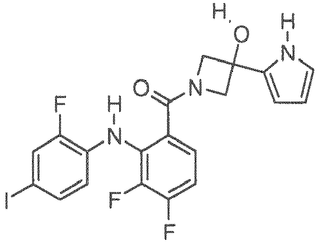 |
| 272 | 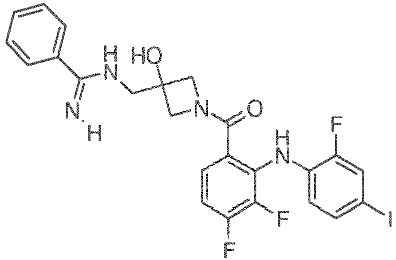 |
| 273 | 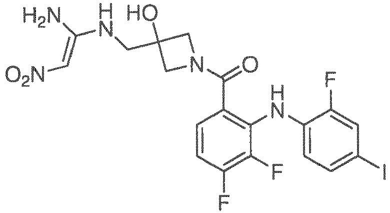 |
| 274 | 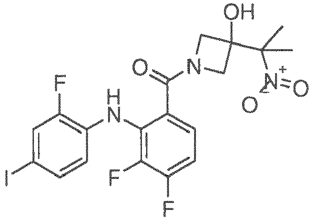 |
| 275 | 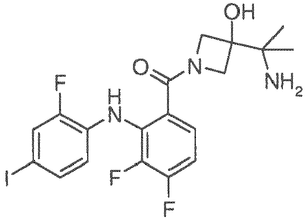 |
| 276 | 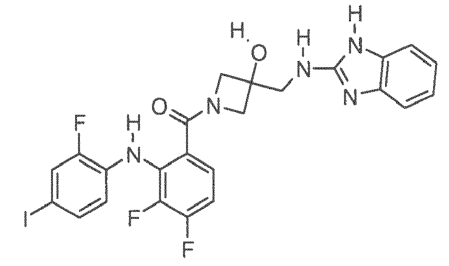 |
| 277 | 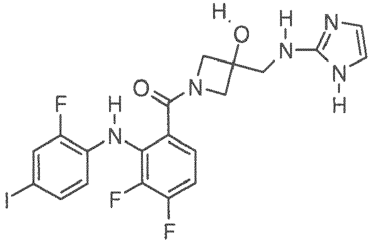 |
| 278 | 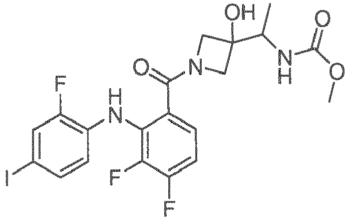 |
| 279 | 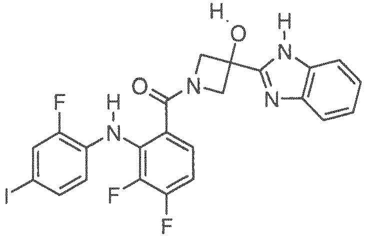 |
| 280 | 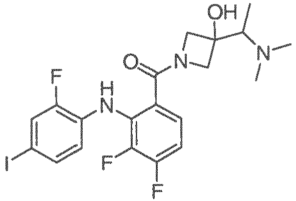 |
| 281 | 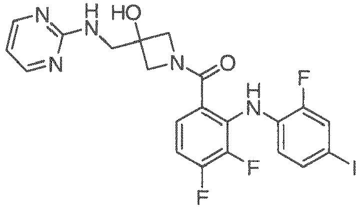 |
| 282 | 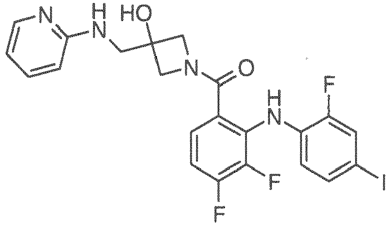 |
| 283 | 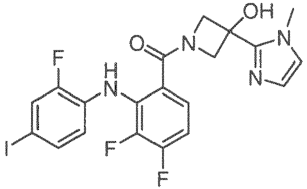 |
| 284 | 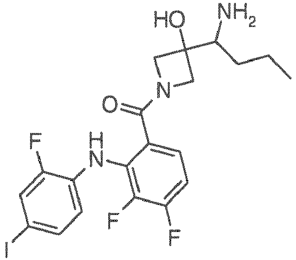 |
| 285 | 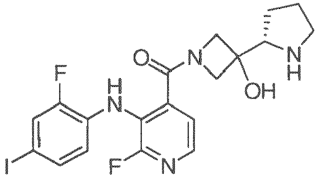 |
| 286 | 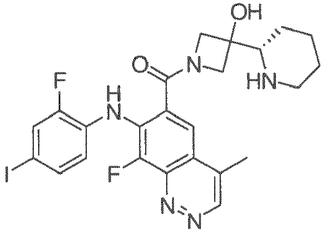 |
| 287 | 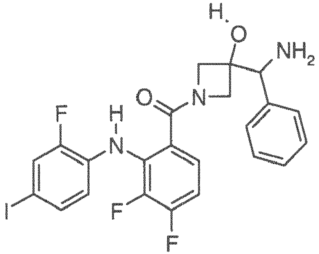 |
| 288 | 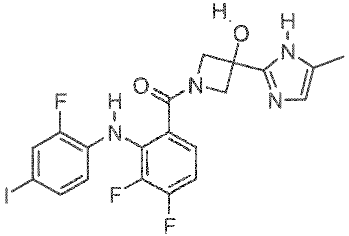 |
| 289 | 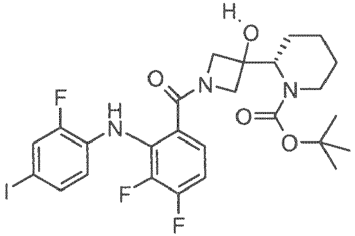 |
| 290 | 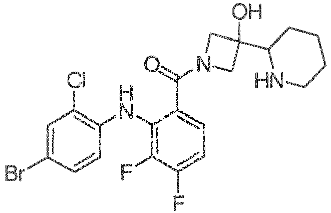 |
| 291 | 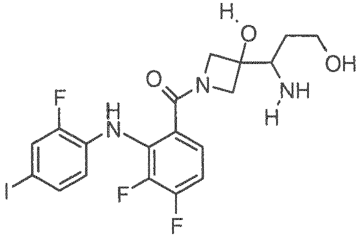 |
| 292 | 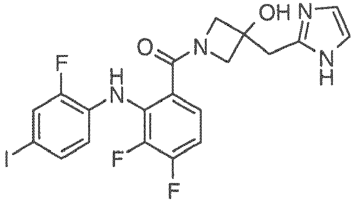 |
| 293 | 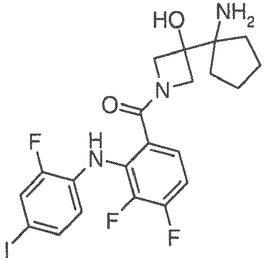 |
| 294 | 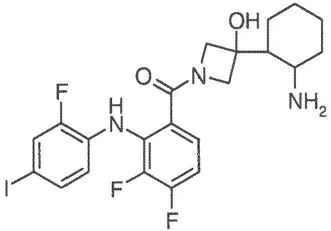 |
| 295 | 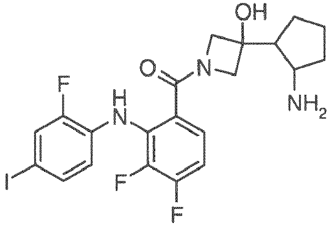 |
| 296 | 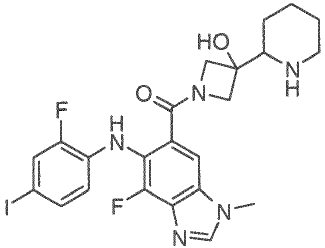 |
| 297 | 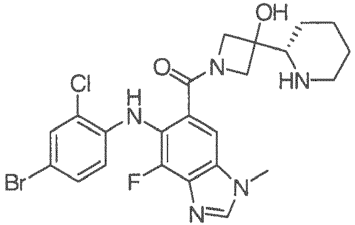 |
| 298 | 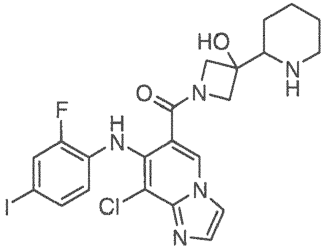 |
| 299 | 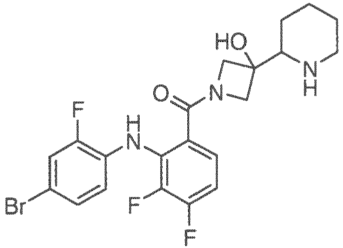 |
| 300 | 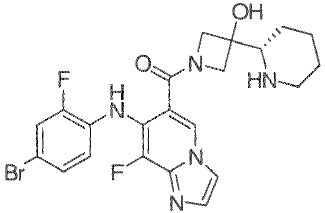 |
| 301 | 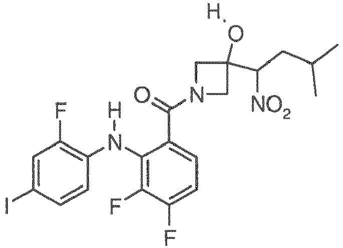 |
| 302 | 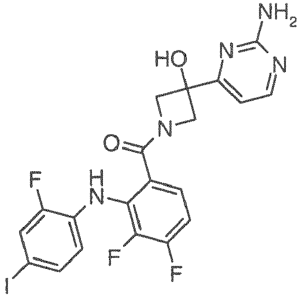 |
| 303 | 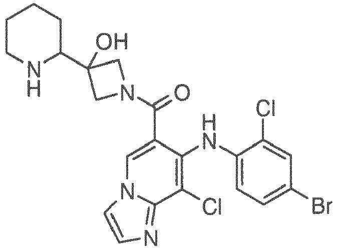 |
| 304 | 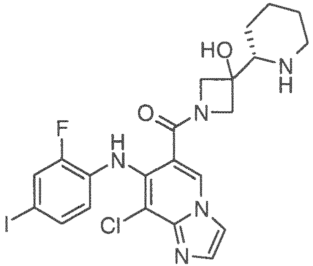 |
| 305 | 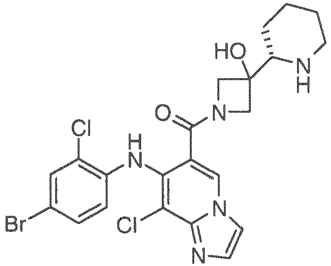 |
| 306 | 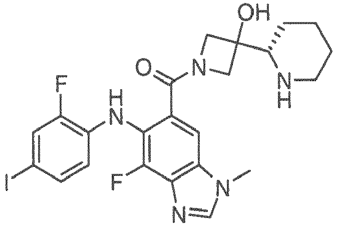 |
| 307 | 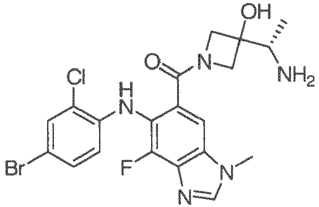 |
| 308 | 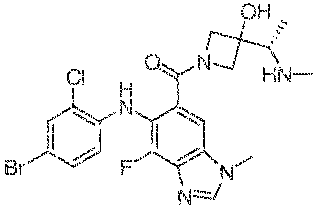 |
| 309 | 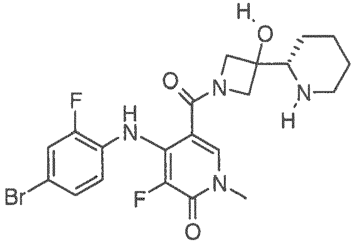 |
| 310 | 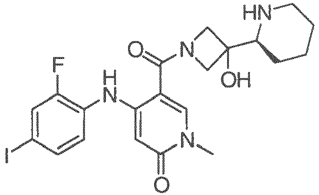 |
| 311 | 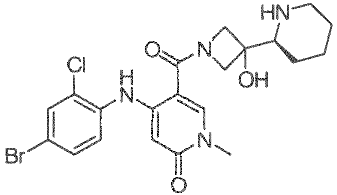 |
| 312 | 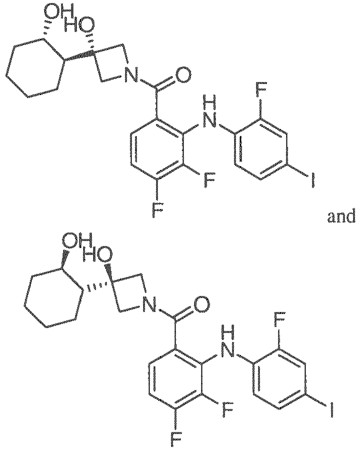 |
| 313 | 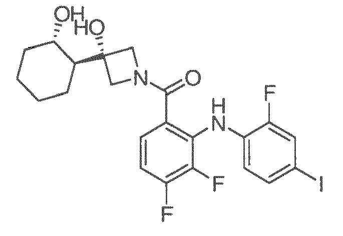 |
| 314 | 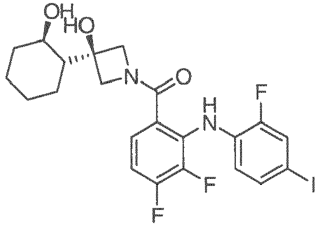 |
| 315 | 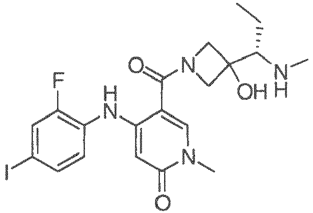 |
| 316 | 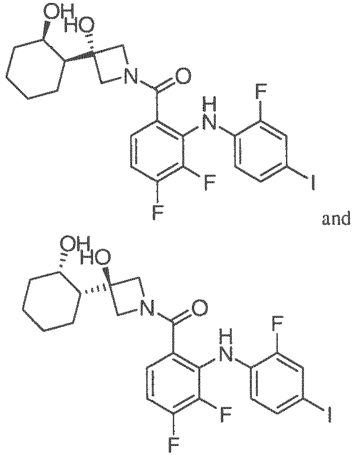 |
| 317 | 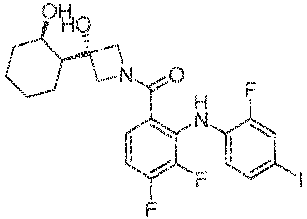 |
| 318 | 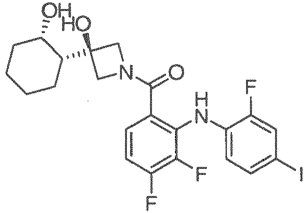 |
| 319 | 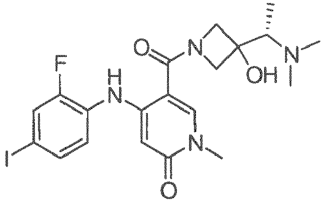 |
| 320 | 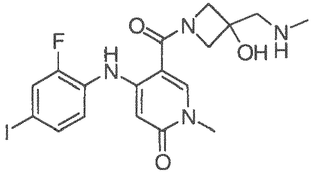 |
| 321 | 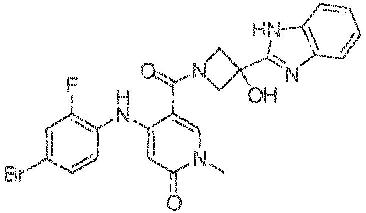 |
| 322 | 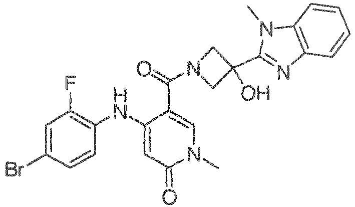 |
| 323 | 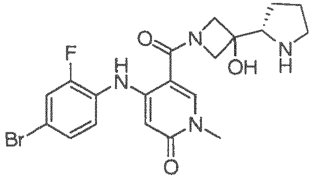 |
| 324 | 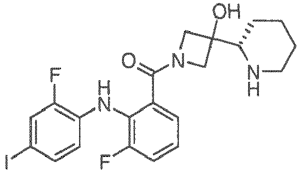 |
| 325 | 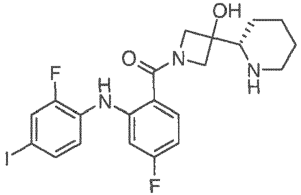 |
| 326 | 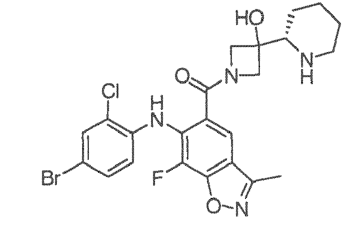 |
| 327 | 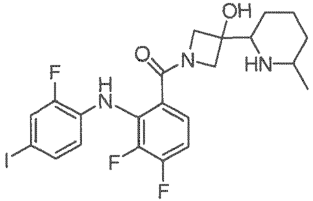 |
| 328 | 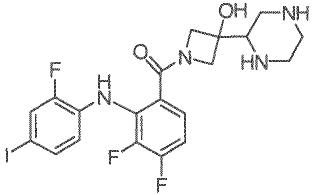 |
| 329 | 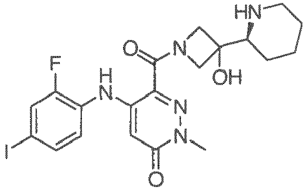 |
| 330 | 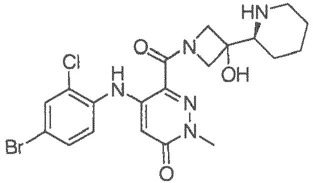 |
| 331 | 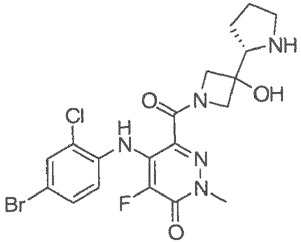 |
| 332 | 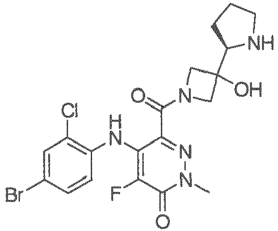 |
| 333 | 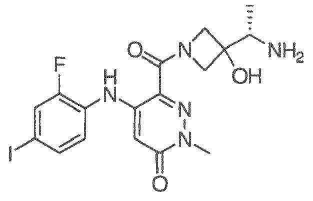 |
| 334 | 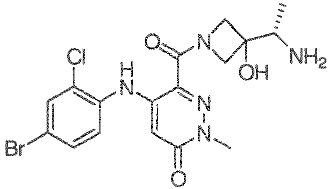 |
| 335 | 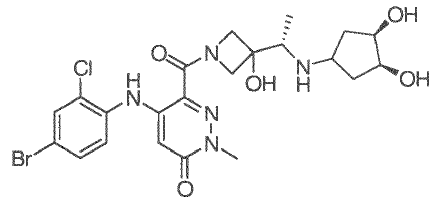 |
| 336 | 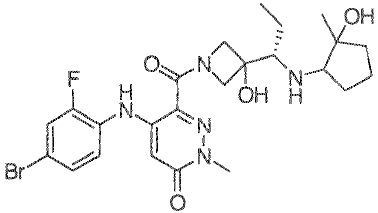 |
| 337 | 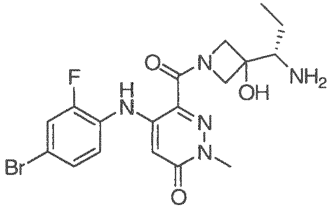 |
| 338 | 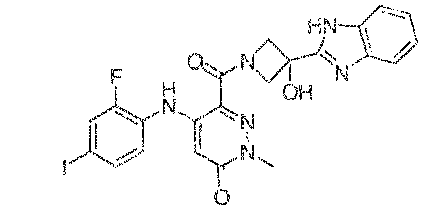 |
| 339 | 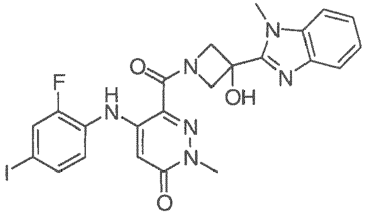 |
| 340 | 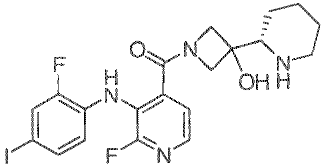 |
| 341 | 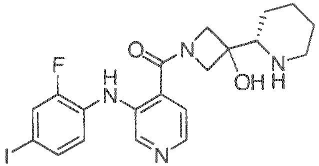 |
| 342 | 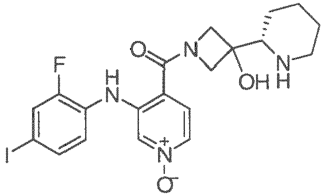 |
| 343 | 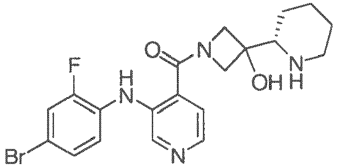 |
| 344 | 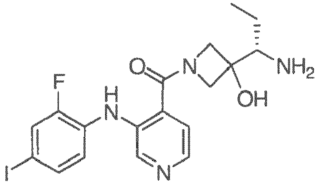 |
| 345 | 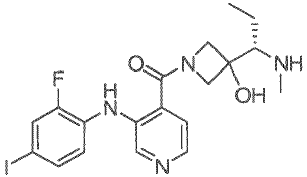 |
| 346 | 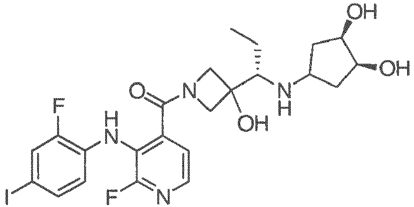 |
| 347 | 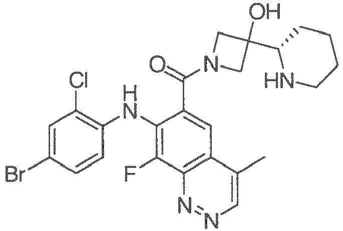 |
| 348 | 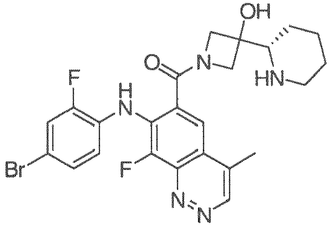 |
| 349 | 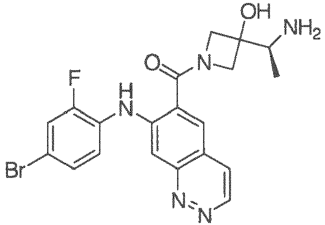 |
| 350 | 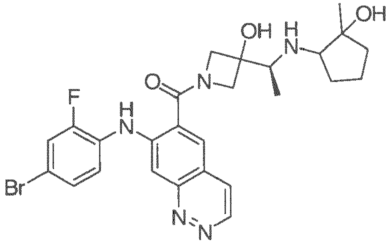 |
| 351 | 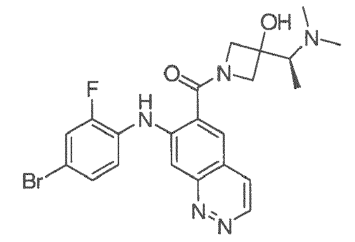 |
| 352 | 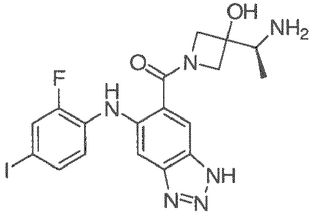 |
| 353 | 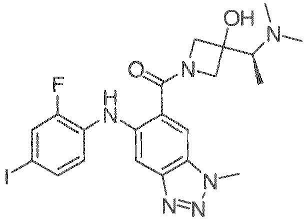 |
| 354 | 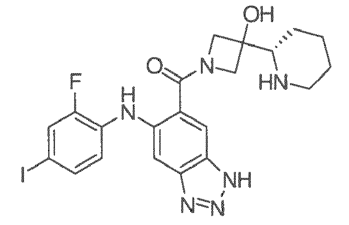 |
| 355 | 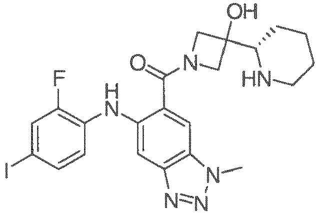 |
| 356 | 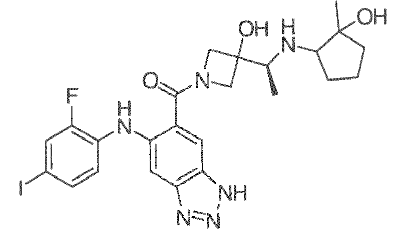 |
| 357 | 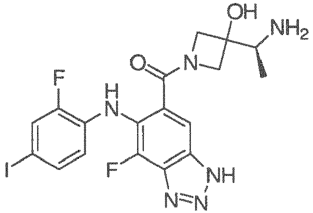 |
| 358 | 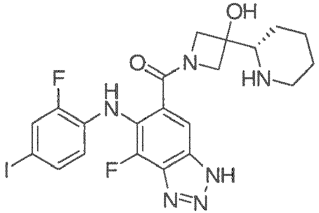 |
| 359 | 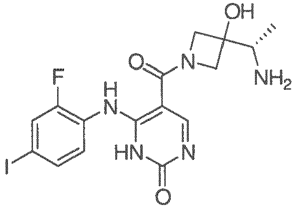 |
| 360 | 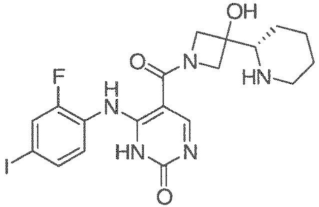 |
| 361 | 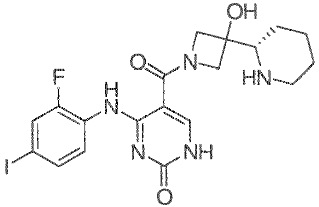 |
| 362 | 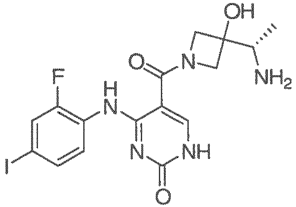 |

RAS/RAF mutational status in patients with locally advanced or metastatic solid tumors may be determined from tumor tissue samples from patients using methods known in the art, e.g. to ascertain the presence or absence of BRaf (e.g., BRaf ^{V600E}), NRas or KRas muations described above.

The PI3K inhibitor GDC-0941 and MEK inhibitors including GDC-0973/XL518 of the present invention may exist in unsolvated as well as solvated forms with pharmaceutically acceptable solvents such as water, and ethanol, and it is intended that the invention embrace both solvated and unsolvated forms.

The PI3K inhibitor GDC-0941 and MEK inhibitors including GDC-0973/XL518 of the present invention may also exist in different tautomeric forms, and all such forms are embraced within the scope of the invention. The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

The PI3K inhibitor GDC-0941 and MEK inhibitors including GDC-0973/XL518 of the present invention may also be isotopically-labeled, i.e., one or more atoms are replaced by an atom having an atomic mass or mass number different from the atomic mass or mass number usually found in nature. All isotopes of any particular atom as specified are contemplated within the scope of the compounds of the invention, and their uses. Exemplary isotopes that can be incorporated into the PI3K inhibitor GDC-0941 and MEK inhibitors including GDC-0973/XL518 include isotopes of hydrogen, carbon, nitrogen, oxygen, phosphorus, sulfur, fluorine, chlorine and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²¹I. Certain isotopically-labeled compounds of the present invention (e.g., those labeled with ³H and ¹⁴C) are useful in compound and/or substrate tissue distribution assays. Tritiated (³H) and carbon-14 (¹⁴C) isotopes are useful for their ease of preparation and detectability. Further, substitution with heavier isotopes such as deuterium (²H) may afford certain therapeutic advantages resulting from greater metabolic stability (e.g., increased in vivo half-life or reduced dosage requirements) and hence may be preferred in some circumstances. Positron emitting isotopes such as ¹⁵O, ¹³N, ¹¹C and ¹⁸F are useful for positron emission tomography (PET) studies to examine substrate receptor occupancy. Isotopically labeled compounds of the present invention can generally be prepared by substituting an isotopically labeled reagent for a non-isotopically labeled reagent.

The PI3K inhibitor GDC-0941 and MEK inhibitors including GDC-0973/XL518 of the present invention may be administered in the form of a pharmaceutical composition comprising GDC-0941 and a pharmaceutical composition comprising a MEK inhibitor including GDC-0973/XL518, wherein said pharmaceutical compositions comprise one or more pharmaceutically acceptable carrier, glidant, diluent, or excipient.

Suitable carriers, diluents and excipients are well known to those skilled in the art and include materials such as carbohydrates, waxes, water soluble and/or swellable polymers, hydrophilic or hydrophobic materials, gelatin, oils, solvents, and water. The particular carrier, diluent or excipient used will depend upon the means and purpose for which the compound of the present invention is being applied. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG 400, PEG 300), etc. and mixtures thereof. The compositions may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug (i.e., a compound of the present invention or pharmaceutical composition thereof) or aid in the manufacturing of the pharmaceutical product (i.e., medicament).

The compositions may be prepared using conventional dissolution and mixing procedures. For example, the bulk drug substance (i.e., compound of the present invention or stabilized form of the compound (e.g., complex with a cyclodextrin derivative or other known complexation agent) is dissolved in a suitable solvent in the presence of one or more of the excipients described above.

The pharmaceutical compositions include those suitable for the administration routes detailed herein. The compositions may conveniently be presented in unit dosage form and may be prepared by any of the methods well known in the art of pharmacy. Techniques and formulations generally are found in Remington's Pharmaceutical Sciences 18th Ed. (1995) Mack Publishing Co., Easton, PA. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Also falling within the scope of this application are methods of treating a patient with locally advanced or metastatic solid tumors with the combination of the *in vivo* metabolic products of GDC-0941 and MEK inhibitors described herein including GDC-0973/XL-518, in accordance to the regimens described above. Such products may result e.g. from the oxidation, reduction, hydrolysis, amidation, deamidation, esterification, deesterification, and enzymatic cleavage.

### EXAMPLES

In order to illustrate the invention, the following examples are included.

### Example 1: In vitro combination efficacy of GDC-0941 and GDC-0973 in a set of melanoma and NSCLC cell lines using a 4-day CellTiterGlo viability assay

Calcusyn, a program utilizing the Chou and Talalay (1984) method of calculating synergy, was used to calculate the combination index and, thus, determine the level of synergy (Figure 1). Strong synergy, as indicated by combination index values ≤ 0.3, was observed in the majority of melanoma cell lines, about 60% of which carry oncogenic mutations in BRAF. Synergy, as indicated by combination index values ≤ 0.7, or strong synergy was observed in all but one of the NSCLC cell lines, of which about half carry oncogenic mutations in KRAS.

### Example 2: GDC-0973 and GDC-0941 as therapeutic agents in NCI-H2122 (NSCLC, KRAS^{G12C}, PI3K/PTEN WT) and A2058 (Melanoma, BRAF^{V600E}, PTEN^{null}) xenograft models

Both of these models show moderate sensitivity to GDC-0973 or GDC-0941 as single agents; however, neither has exquisite sensitivity to either drug alone, resulting in tumor growth delay, but not stasis or regression. Therefore, pathway activity for both MEK and PI3K is evident in each model, making these relevant models to test combinations of MEK and PI3K inhibitors. NCI-H2122 tumor-bearing animals were treated with GDC-0973 (5 mg/kg, daily), GDC-0941 (50 mg/kg, daily) or the combination. NCI-H2122 tumors showed sensitivity to single agent GDC-0973 with tumor growth inhibition (TGI, relative to vehicle control) of 57% in the 5 mg/kg QD arm (Figure 2). Likewise, NCI-H2122 tumors are also sensitive to single agent GDC-0941 with a TGI of 73% in the 30 mg/kg arm (Figure 2). The combination of GDC-0973 and GDC-0941 resulted in a marked improvement in efficacy over either single agent at both dose levels, resulting in 98% TGI, or tumor stasis (Figure 2). Student's t-tests comparing the moderate doses of GDC-0973 or GDC-0941 to the combination of the two agents revealed that the anti-tumor effect was statistically significant (Student's t-test p = 0.032, p = 0.046, respectively, on Day 21; Figure 2). GDC-0973 and GDC-0941 were well tolerated when administered alone and in combination, even at higher doses of GDC-0973 at 10 mg/kg, daily with GDC-0941 at 100 mg/kg, daily (data not shown). These data show that GDC-0973 and GDC-0941 have a significant impact on tumor growth in the NCI-H2122 NSCLC xenograft model when used in combination.

### Example 3: Moderate and high doses of GDC-0973 (6 and 10 mg/kg, daily) and GDC-0941 (30 and 100 mg/kg, daily) as well as combinations of the lower and higher doses in A2058 tumor-bearing animals

Similar to NCI-H2122, A2058 tumors also show moderate sensitivity to single agent GDC-0973 with TGIs of 41% at 6 mg/kg and 62% at 10 mg/kg (Figure 3A and B, respectively). Likewise, A2058 tumors are also moderately sensitive to single agent GDC-0941 with TGIs of 18% at 30 mg/kg and 56% at 100 mg/kg (Figure 3A and B, respectively). The combination of GDC-0973 and GDC-0941 results in a marked improvement in efficacy over either single agent, resulting in an improvement to 69% TGI at lower doses and 90% TGI at higher doses, approaching tumor stasis (Figure 3A and B, respectively). Student's t-tests comparing the moderate doses of GDC-0973 or GDC-0941 to the combination of the two agents revealed that the anti-tumor effect was statistically significant (p = 0.048, p = 0.008, respectively, on Day 17). Similarly, comparison of the high doses of GDC-0973 or GDC-0941 to the combination revealed a significant difference (Student's t-test p = 0.001, p = 0.004, respectively, on Day 17). Combination of GDC-0973 and GDC-0941 did not result in any obvious adverse effects for the mice.

### Example 4: Treatment of FaDu (HNSCC) tumor-bearing animals with either single agent GDC-0973 or GDC-0941 or the combination of the two

FaDu tumors show moderate sensitivity to single agent GDC-0973 at 5 mg/kg, daily with a percent tumor growth inhibition (%TGI) of 41% (Fig. 4A). Similarly, FaDu tumors show moderate response to GDC-0941 at 100 mg/kg with a %TGI of 33% (Figure 4A). The combination of GDC-0973 and GDC-0941 at these same doses results in a marked improvement in efficacy over either single agent, resulting in an improvement in %TGI to 96% TGI (Figure 4A). The improved efficacy of the combination was statistically significant versus single agent GDC-0973 and GDC-0941 (p = 0.0281 and p = 0.0034, respectively, on Day 18).

### Example 5: Treatment of SKOV-3 (Ovarian) tumor-bearing animals with either single agent GDC-0973 (10 mg/kg, daily) or GDC-0941 (100 mg/kg, daily) or the combination of the two

SKOV-3 tumors show moderate response to GDC-0941 at 100 mg/kg with a %TGI of 51 % (Fig. 4B). The combination of GDC-0973 and GDC-0941 at these same doses results in a marked improvement in efficacy over either single agent, resulting in an improvement in %TGI to 91% TGI (Fig. 4B). The improved efficacy of the combination was statistically significant versus single agent GDC-0973 and GDC-0941 (p = 0.0017 and p = 0.0283, respectively, on Day 22).

### Example 6: Treatment of MOLM-16 (AML) tumor-bearing animals with either single agent GDC-0973 or GDC-0941 or the combination of the two

MOLM-16 tumors show sensitivity to single agent GDC-0973 at 10 mg/kg, daily with a percent tumor growth inhibition (%TGI) of 57% (Fig. 5). Similarly, MOLM-16 tumors show moderate response to GDC-0941 at 100 mg/kg with a %TGI of 40% (Figure 5). The combination of GDC-0973 and GDC-0941 at these same doses results in a marked improvement in efficacy over either single agent, resulting in an improvement in %TGI to 96% TGI (Figure 5). The improved efficacy of the combination was statistically significant versus single agent GDC-0973 and GDC-0941 (p = 0.0381 and p = 0.0158, respectively, on Day 11).

### Example 7: Safety and tolerability study of GDC-0973 and GDC-0941 when administered in combination in patients with locally advanced or metastatic solid tumors

In this study, daily oral dosing of GDC-0973 (5mg or 25mg capsules) and GDC-0941 (15mg or 50mg capsules) administered in combination, daily for 21 days followed by 7 days without study drug, in patients with locally advanced or metastatic solid tumors that are RAS/RAF wild-type, mutant, or unknown. Archival tumor specimens will be obtained from all patients to confirm or determine BRAF, NRAS or KRAS mutational status and PI3K mutation/PI3K amplification/- PTEN protein status. All patients will have serial FDG-PET imaging as PD biomarker and potential early readout of anti-tumor activity. Treatment will continue for up to 1 year or until disease progression, unacceptable toxicity or any other discontinuation criterion is met. Concurrent GDC-0973 and GDC-0941 administration will be QD (once daily) for 21 consecutive days of a 28-day cycle according to one of the following dosing schema in Figures 6a and 6b. Alternate dosing regimens and schedules may be 14 days on both GDC-0973 and GDC-0941 followed by 14 days off both GDC-0973 and GDC-0941 or intermittent dosing schedules (such as Q2D (every 2 days) and Q4D (every 4 days). Tables 1-A and 1-B show alternative treatment schedule for concurrent oral administration of GDC-0973 and GDC-0941.

Additional patients with RAS/RAF mutant locally advanced or metastatic solid tumors who have had no more than four prior systemic therapies (for their locally advanced or metastatic cancer) will be enrolled to gather additional safety, PK and PD data at one of the treatment schedules in Tables 1-A and 1-B and dosing schema of Figures 6a and 6b. Pre- and post-treatment tumor biopsy samples for PD biomarker analyses will be collected from all patients. All patients will have serial FDG-PET imaging as a potential early readout of anti-tumor activity. Mutational status will be determined retrospectively from mandatory collection of archival tumor tissue samples.

| **Table 1-A** Treatment Schedule | | | **Table 1-B** Treatment Schedule | | |
|---|---|---|---|---|---|
| Group | GDC-0973 | GDC-0941 | Group | GDC-0973 | GDC-0941 |
| 1 | 20 mg | 80 mg | | | |
| 2 | 20 mg | 100 mg | 2a | 20 mg | 130 mg |
| 3 | 40 mg | 80 mg | 3a | 60 mg | 80 mg |
| 4 | 40 mg | 100 mg | | | |
| 5 | 40 mg | 130 mg | 5a | 40 mg | 180 mg |
| 6 | 60 mg | 100 mg | | | |
| 7 | 60 mg | 130 mg | 7a | 60 mg | 180 mg |

Disease status will be assessed using Response Evaluation Criteria in Solid Tumors (RECIST) (Therasse et al., New guidelines to evaluate the response to treatment in solid tumors. J Natl Cancer Inst 2000;92:205-16). Tumor status will be categorized as complete response, partial response, stable disease, or progressive disease per RECIST. Objective response will be confirmed by repeat physical examination or image-based evaluation ≥4 weeks after the initial documentation, per RECIST.

## Claims

1. Therapeutic combination of a therapeutically effective amount of a compound of formula **I,** or a pharmaceutically acceptable salt of **I**, and a therapeutically effective amount of a compound of formula **III,** or a pharmaceutically acceptable salt of **III.** 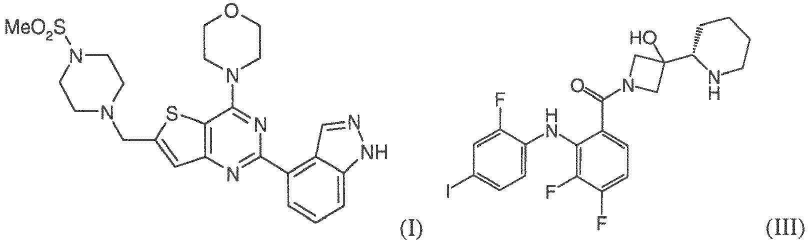 for use in treating locally advanced or metastatic solid tumors in a mammal as a combined formulation or by alternation.

2. Therapeutic formulation of a therapeutically effective amount of a compound of formula I, or a pharmaceutically acceptable salt of **I**, and a therapeutically effective amount of a compound of formula **III,** or a pharmaceutically acceptable salt of **III.** 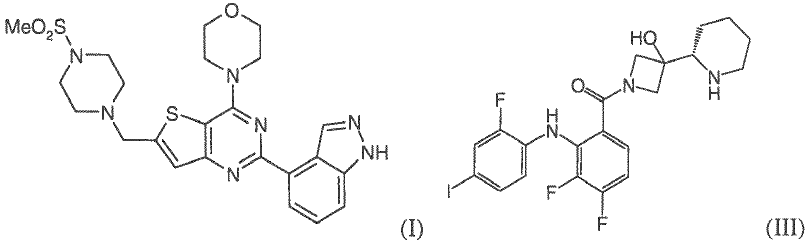 for use in treating locally advanced or metastatic solid tumors in a mammal as a combined formulation or by alternation.

3. Therapeutic formulation for use according to claim 2 wherein said patient is administered concurrently 80 mg, 100 mg, 130 mg or 180 mg of **I** and 20 mg, 40 mg, or 60 mg of **III.**

4. Therapeutic formulation for use according to claim 2 wherein said patient is administered a compound of formula **I** in combination with a compound of formula **III** wherein said patient is on a 28 day cycle in which said patient is administered both the compound of formula **I** and the compound of formula **III** for 21 consecutive days and no compound of formula **I** or **III** for the next 7 consecutive days.

5. Therapeutic formulation for use according to claim 2 wherein said patient is administered a compound of formula **I** in combination with a compound of formula **III** wherein said patient is on a 28 day cycle in which said patient is administered both, the compound of formula **I** and the compound of formula **III** for 14 consecutive days and no compound of formula **I or III** for the next 14 consecutive days.

6. Use of a compound of formula I in combination with a compound of formula **III** 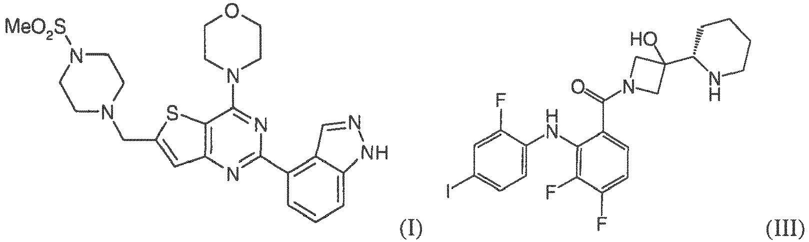 in the preparation of a medicament for the treatment of locally advanced or metastatic solid tumors.

## Patentansprüche

1. Therapeutische Kombination einer therapeutisch wirksamen Menge einer Verbindung der Formel **I,** oder eines pharmazeutisch verträglichen Salzes von I, und einer therapeutisch wirksamen Menge einer Verbindung der Formel **III,** oder eines pharmazeutisch verträglichen Salzes von **III.** 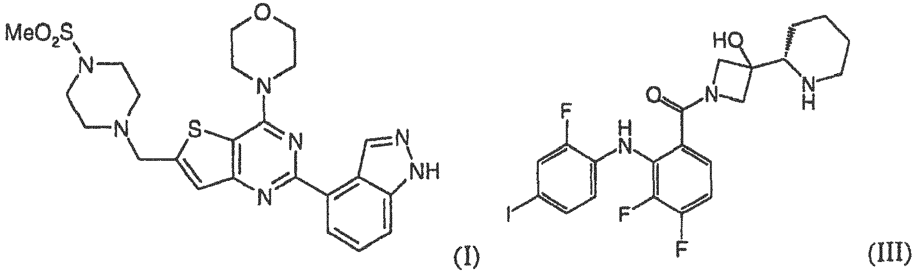 zur Verwendung bei der Behandlung von lokal fortgeschrittenen oder metastatisierenden festen Tumoren in einem Säuger, als eine kombinierte Formulierung oder durch Wechsel.

2. Therapeutische Formulierung einer therapeutisch wirksamen Menge einer Verbindung der Formel **I,** oder eines pharmazeutisch verträglichen Salzes von **I,** und einer therapeutisch wirksamen Menge einer Verbindung der Formel **III,** oder eines pharmazeutisch verträglichen Salzes von **III.** 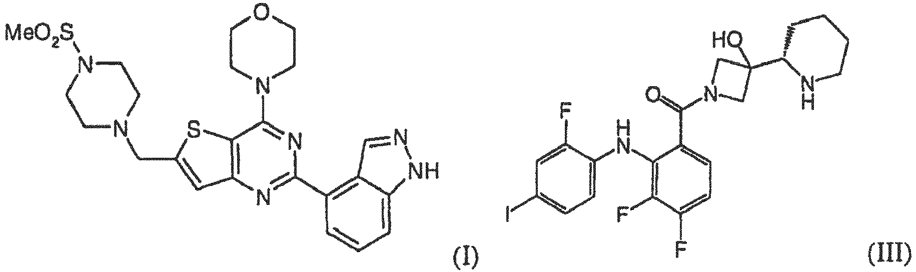 zur Verwendung bei der Behandlung von lokal fortgeschrittenen oder metastatisierenden festen Tumoren in einem Säuger, als eine kombinierte Formulierung oder durch Wechsel.

3. Therapeutische Formulierung zur Verwendung gemäß Anspruch 2, wobei dem Patienten 80 mg, 100 mg, 130 mg oder 180 mg von **I** und 20 mg, 40 mg oder 60 mg von **III** zugleich verabreicht werden.

4. Therapeutische Formulierung zur Verwendung gemäß Anspruch 2, wobei dem Patienten eine Verbindung der Formel **I** in Kombination mit einer Verbindung der Formel **III** verabreicht wird, wobei sich der Patient in einem 28-Tage-Zyklus befindet, in welchem dem Patienten sowohl die Verbindung der Formel **I** als auch die Verbindung der Formel **III** für 21 aufeinanderfolgende Tage und keine Verbindung der Formel **I** oder **III** für die nächsten 7 aufeinanderfolgende Tage verabreicht werden.

5. Therapeutische Formulierung zur Verwendung gemäß Anspruch 2, wobei dem Patienten eine Verbindung der Formel **I** in Kombination mit einer Verbindung der Formel **III** verabreicht wird, wobei sich der Patient in einem 28-Tage-Zyklus befindet, in welchem dem Patienten sowohl die Verbindung der Formel **I** als auch die Verbindung der Formel **III** für 14 aufeinanderfolgende Tage und keine Verbindung der Formel **I** oder **III** für die nächsten 14 Tage verabreicht werden.

6. Verwendung einer Verbindung der Formel **I** in Kombination mit einer Verbindung der Formel **III** 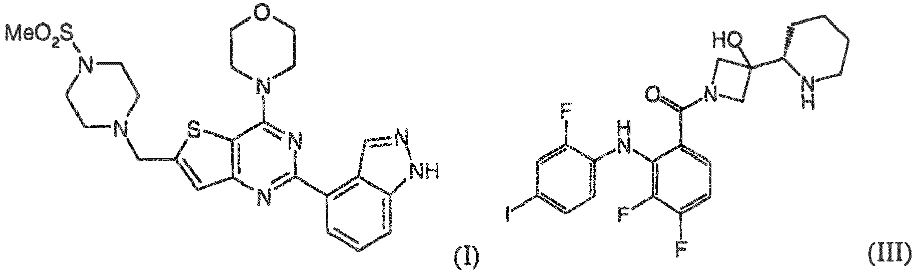 bei der Herstellung eines Medikaments zur Behandlung von lokal fortgeschrittenen oder metastatisierenden festen Tumoren.

## Revendications

1. Association thérapeutique d'une quantité thérapeutiquement efficace d'un composé de formule (I), ou d'un sel pharmaceutiquement acceptable de (I) et d'une quantité thérapeutiquement efficace d'un composé de formule (III), ou d'un sel pharmaceutiquement acceptable de (III) 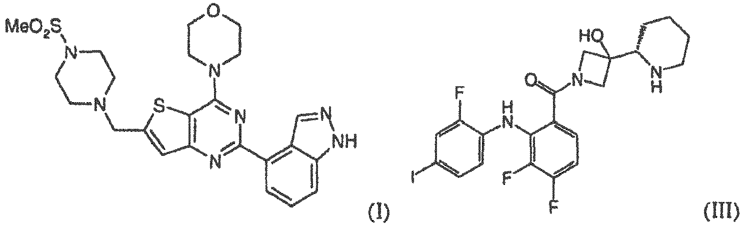 pour son utilisation dans le traitement de tumeurs solides locales de stade avancé ou métastatiques chez un mammifère sous forme de formulation combinée ou en alternance.

2. Formulation thérapeutique d'une quantité thérapeutiquement efficace d'un composé de formule (I), ou d'un sel pharmaceutiquement acceptable de (I) et d'une quantité thérapeutiquement efficace d'un composé de formule (III), ou d'un sel pharmaceutiquement acceptable de (III) 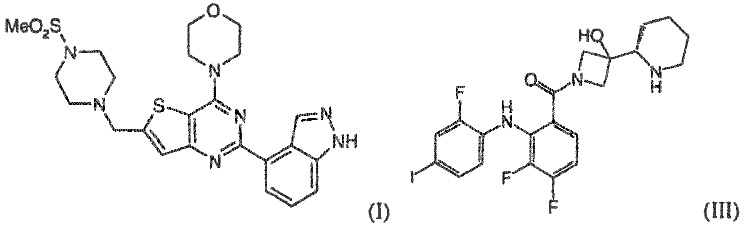 pour son utilisation dans le traitement de tumeurs solides locales de stade avancé ou métastatiques chez un mammifère sous forme de formulation combinée ou en alternance.

3. Formulation thérapeutique pour son utilisation selon la revendication 2, dans laquelle ledit patient reçoit de manière simultanée 80 mg, 100 mg, 130 mg ou 180 mg de (I) et 20 mg, 40 mg ou 60 mg de (III).

4. Formulation thérapeutique pour son utilisation selon la revendication 2, dans laquelle ledit patient reçoit un composé de formule (I) en association avec un composé de formule (III), ledit patient étant sur un cycle de 28 jours pendant lesquels ledit patient reçoit à la fois le composé de formule (I) et le composé de formule (III) pendant 21 jours consécutifs et pas de composé de formule (I) ou (III) pendant les 7 jours consécutifs suivants.

5. Formulation thérapeutique pour son utilisation selon la revendication 2, dans laquelle ledit patient reçoit un composé de formule (I) en association avec un composé de formule (III), ledit patient étant sur un cycle de 28 jours pendant lesquels ledit patient reçoit à la fois le composé de formule (I) et le composé de formule (III) pendant 14 jours consécutifs et pas de composé de formule (I) ou (III) pendant les 14 jours consécutifs suivants.

6. Utilisation d'un composé de formule (I) en association avec un composé de formule (III) 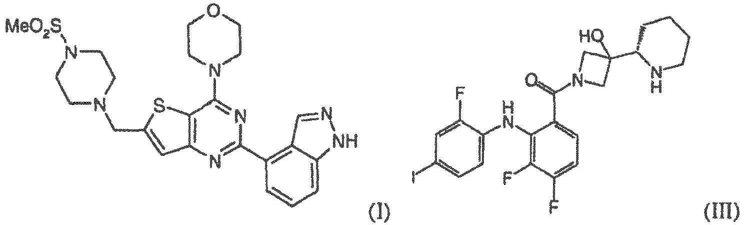 dans la préparation d'un médicament destiné au traitement de tumeurs solides locales de stade avancé ou métastatiques.
